# EUROPÄISCHE PATENTSCHRIFT

(11) **EP 1 275 711 B1**
(45) Veröffentlichungstag und Bekanntmachung des Hinweises auf die Patenterteilung: **12.05.2010**
(21) Anmeldenummer: 01401855.0
(22) Anmeldetag: 11.07.2001
(51) Int. Cl.: C12N 9/20, C12N 15/62, C12N 15/55, C12N 1/19, C12N 1/21

(54) **Lipase/Acyltransferase aus Candida parapsilosis**
Lipase/acyltransferase from Candida parapsilosis
Lipase/acyltransferase de Candida parapsilosis

(43) Veröffentlichungstag der Anmeldung: 15.01.2003
(73) Patentinhaber: Cognis IP Management GmbH, 40589 Düsseldorf (DE)
(72) Erfinder: Dubreucq, Eric, 34000 Montpellier (FR); Bigey, Frederic, 34070 Montpellier (FR); Moulin, Guy, 34980 Montferrier-sur-Lez (FR); Weiss, Albrecht Dr., 40764 Langenfeld (DE)

(56) Entgegenhaltungen:
- DATABASE TREMBL [Online] Acc.Nos. Q9P8W6/AF188894 & Q9P8W1/AF191317, 1. Oktober 2000 (2000-10-01) HUBE ET AL.: "Secreted lipases of Candida albicans: Cloning and characterization of a new group of putative virulence genes " XP002185267
- HUBE BERNHARD ET AL: "Secreted lipases of Candida albicans: Cloning, characterisation and expression analysis of a new gene family with at least ten members." ARCHIVES OF MICROBIOLOGY, Bd. 174, Nr. 5, November 2000 (2000-11), Seiten 362-374, XP002185265 ISSN: 0302-8933
- VAYSSE L ET AL: "Fatty hydroxamic acid biosynthesis in aqueous medium in the presence of the lipase-acyltransferase from Candida parapsilosis" JOURNAL OF BIOTECHNOLOGY, ELSEVIER SCIENCE PUBLISHERS, AMSTERDAM, NL, Bd. 53, Nr. 1, 28. Februar 1997 (1997-02-28), Seiten 41-46, XP004094622 ISSN: 0168-1656
- DATABASE TREMBL [Online] Accession No. U34807, 6. Januar 1999 (1999-01-06) FU ET AL.: "Lip1_canal Lipase 1 (EC 3.1.1.3.)" XP002185269
- FU YUE ET AL: "Cloning and characterization of a gene (LIP1) which encodes a lipase from the pathogenic yeast Candida albicans." MICROBIOLOGY (READING), Bd. 143, Nr. 2, 1997, Seiten 331-340, XP001037888 ISSN: 1350-0872
- BRIAND DELPHINE ET AL: "Substrate specificity of the lipase from Candida parapsilosis." LIPIDS, Bd. 30, Nr. 8, 1995, Seiten 747-754, XP001036850 ISSN: 0024-4201
- DATABASE TREMBL [Online] Accession No. O94015, 1. Mai 1999 (1999-05-01) TAIT ET AL.: "Hypothetical 49.9kDa protein" XP002185268
- TAIT EVELYN ET AL: "A Candida albicans genome project: Cosmid contigs, physical mapping and gene isolation." FUNGAL GENETICS AND BIOLOGY, Bd. 21, Nr. 3, 1997, Seiten 308-314, XP002185266 ISSN: 1087-1845

## Beschreibung

### Gebiet der Erfindung

Die vorliegende Erfindung bezieht sich auf Polypeptide mit Lipase/Acyltransferase Aktivität, Aminosäuresequenzen von Polypeptiden, die diese Aktivität zeigen, auf Nucleinsäuren (Gene), die für diese Polypeptide kodieren, auf Vektoren, die Nucleinsäuren enthalten, welche für diese Polypeptide kodieren, auf transformierte Mikroorganismen die diese Nucleinsäuren enthalten, auf Verfahren zur Herstellung dieser Polypeptide sowie auf die Verwendung von Nucleinsäuren zum Auffinden neuer Lipase/Acyltransferasen und auf die Verwendung dieser Lipase/Acyltransferasen als Katalysatoren in chemischen und biochemischen Verfahren.

### Stand der Technik

Bei der Veresterung nach den üblichen Methoden der chemischen Synthese kann es einerseits wegen der Anwesenheit mehrerer frei vorliegender Hydroxylgruppen in der alkoholischen Komponente oder einem Partialester hiervon in der Regel zur Bildung von Gemischen aus einfach und mehrfach substituierten Produkten kommen, so dass die Einführung und Entfernung von Schutzgruppen notwendig ist, wenn man gezielt eine bestimmte Verbindung synthetisieren will.

Durch den Einsatz aktivierter Carbonsäurederivate entstehen Beiprodukte und häufig auch unerwünschte Nebenprodukte, welche die Aufarbeitung erschweren, die Ausbeuten an gewünschtem Produkt vermindern und die Umwelt belasten. Diese Nachteile können vermieden oder zumindest vermindert werden, indem die Herstellung auf enzymatischem Weg (z. B. in Anlehnung an das in der deutschen Anmeldung DE 197 53 789.8 beschriebene Verfahren) erfolgt.

In der chemischen und biochemischen Synthese werden vermehrt Enzyme als Katalysatoren eingesetzt. So werden in vielen Fällen aufgrund der oft milderen Reaktionsbedingungen bereits in großtechnischen Verfahren Hydrolasen, speziell Lipasen (EC 3.1.1.3) zur Fettspaltung eingesetzt.

Geeignete enzymatische Verfahren zur Veresterung oder Umesterung sind beispielsweise beschrieben in K. Drauz und H. Waldmann, Enzyme Catalysis in Organic Synthesis, VCH-Verlag, Weinheim 1975.

Bekannt ist, dass Transesterifikation in wasserfreien Medien durch Lipasen katalysiert werden. Ist im Reaktionssystem von Estern, Alkohol und Lipasen auch Wasser vorhanden, so setzt normalerweise eine Abspaltung von gebundenen Fettsäuren zu freien Fettsäuren ein. Da verschiedene Lipasen auch die Bildung von Estern aus freien Fettsäuren und Alkoholen katalysieren, werden im Endeffekt eine Transesterifikationsreaktionen mit einer Säurezwischenstufe durchgeführt. Für viele technische Prozesse ist allerdings von großem Nachteil, dass freie Säuren im System gebildet werden. Teilweise verhindert der Wassergehalt eine technisch und kommerziell akzeptable Umsetzung (Ausbildung eines unvorteilhaften thermodynamischen Gleichgewichts). Aufwendige technische Anlagen (Wasserentfernung über z.B. azeotrope Destillation, Membrantrennverfahren, Vakuumdestillation) müssen zur Erzielung befriedigender Ausbeuten eingesetzt werden.

In der Literatur wurde bereits ein bisher einzigartiges Polypeptid beschrieben, welches Lipase/Acyltransferase Aktivität zeigt. Dieses Polypeptid wurde isoliert aus dem Mikroorganismus *Candida parapsilosis* CBS 604. Bisher wurde jedoch ausschließlich die Reaktionsfähigkeit dieses Polypeptids beurteilt. Mit dem Polypeptid aus dem Mikroorganismus *Candida parapsilosis,* das sowohl Lipase als auch Acyltransferase-Eigenschaften zeigt, ist es möglich, die Zwischenstufe einer freien (Fett)säure auch in Gegenwart von Wasser (mit einer Aktivität > 0,8) sehr niedrig zu halten. Gleichzeitig lässt sich der technische Aufwand in Grenzen halten. (vergleiche L. Vaysse, E. Dubreucq, J.-L. Pirat, P. Galzy; J. Biotech. 53 (1997) 41-46).

Der Nachteil bei enzymatisch katalysierten Reaktionen liegt oft in der Verfügbarkeit und Stabilität der Polypeptide.

### Beschreibung der Erfindung

Die Aufgabe der vorliegenden Patentanmeldung hat darin bestanden, Polypeptide zu charakterisieren und zur Verfügung zu stellen, die auch in wässrigem Milieu einen Acyltransfer in hohen Ausbeuten ermöglichen und damit die herkömmlichen Nachteile bei einer Lipase-katalysierten Veresterung überwinden.

Die Aufgabe der vorliegenden Patentanmeldung hat also darin bestanden, diese Polypeptide zu isolieren, die Aminosäuresequenz und die Nucleotidsequenz, die für diese Aminosäuresequenz kodiert, zu entschlüsseln da diese sowohl für die biotechnologische Herstellung als auch für die Weiterentwicklung des Polypeptides unerlässlich sind.

Eine weitere Teilaufgabe bestand darin, die biotechnologische Produktion der gefundenen Lipase/Acyltransferase zu ermöglichen und dadurch in höheren Ausbeuten zur Verfügung zu stellen und dann die Möglichkeit zu besitzen, über ein Screening Sequenzhomologien in alternativen Organismen aufzufinden, Mit eingeschlossen in diese Aufgabe war damit, transformierte Wirtszellen bereitzustellen, die in der Lage sind, dieses Polypeptid zu produzieren.

Unter einem Polypeptid ist im Sinne der vorliegenden Anmeldung ein aus den natürlichen Aminosäuren zusammengesetztes, weitgehend linear aufgebautes, zur Ausübung seiner Funktion zumeist dreidimensionale Struktur annehmendes Polymer zu verstehen. In der vorliegenden Anmeldung werden die 19 proteinogenen, natürlich vorkommenden L-Aminosäuren mit den international gebräuchlichen 1- und 3-Buchstaben-Codes bezeichnet. Eine andere Bezeichnung ist auch Protein, wobei es eine Limitierung der Anzahl der Monomereinheiten im Polypeptid von mindestens 50 geben soll.

Im Sinne der Erfindung versteht man unter "Lipase/Acyltransferase Aktivität" die Aktivität eines Polypeptides oder Enzymes, die die Eigenschaften von Lipasen mit Eigenschaften von Acyltransferasen vereinigt. **Lipasen** (EC 3.1.1.3)zählen zur Gruppe der Hydrolasen (spezieller der Esterasen) die spezifisch Fette (Triglyceride) in Glycerin und Fettsäuren spalten; dieser Lipolyse genannte Vorgang spielt sich an der Phasengrenze zwischen Fett und Wasser ab. Eine wichtige Eigenschaft die zur Einstufung in die Gruppe der Hydrolasen führt, ist die Grenzflächenaktivität von Lipasen. Mechanistisch spielt bei der Katalyse eine katalytische Triade aus Serin, Histidin und Asparaginsäure (oder Glutaminsäure) eine Rolle. **Acyltransferasen** (EC 2.3) werden auch als Transacylasen bezeichnet und zählen zur Gruppe der Transferasen. Sie übertragen ganz allgemein Acyl oder speziell Acetylgruppen von einem Donor- zu einem Akzeptormolekül und sind deshalb von besonderer Wichtigkeit beim Auf- und Abbau von Fetten. Studien an der erfindungsgemäßen **Lipase/Acyltransferase** haben gezeigt, dass dieses Polypeptid grenzflächenaktiv ist und Reaktionen katalysiert, die charakteristisch für Lipasen sind. Es wurde weiterhin gefunden, dass dieses Polypeptid in der Lage ist, Transesterifizierungen zu katalysieren bei einem Wassergehalt im Reaktionsgemisch, der einer Wasser-Aktivität größer 0,8 entspricht. Bei diesem Gehalt an Wasser würde eine herkömmliche Lipase nur die Hydrolyse der Ester katalysieren. Es handelt sich also um ein Polypeptid, welches sowohl charakteristische Merkmale für Lipasen als auch für Acyltransferasen zeigt. Bei dem erfindungsgemäßen, natürlich vorkommendem Polypeptid handelt es sich aufgrund von Sequenzhomologien zu bislang bekannten Enzymen wie beispielsweise Lipase aus *Candida albicans* um eine Lipase und aufgrund seiner enzymatischen Aktivität um eine Acyltransferase.

Als bevorzugte Donoren im Sinne der Erfindung für katalytische Reaktionen mit der erfindungsgemäßen Lipase/Acyltransferase kommen alle möglichen Ester, Fette, Triglyceride, 1,3-Diglyceride. 1,2-Diglyceride und 1-Monoglyceride zur Anwendung. Als bevorzugte Acceptoren im Sinne der Erfindung für katalytische Reaktionen mit der erfindungsgemäßen Lipase/Acyltransferase kommen primäre und sekundäre Alkohole mit ein bis fünf Kohlenstoffatomen, insbesondere Ethanol, Propanol, Butanol, 1,2-Propandiol, 1,3-Propandiol, 2-Methyl-1-propanol, 2-Methyl-1-butanol, 3-Methyl-1-butanol und Hydroxylamine zur Anwendung.

Der hier verwendete Ausdruck "Identität" in Bezug auf die Aminosäuresequenz bezeichnet eine Homologie zur gegebenen Aminosäuresequenz die dazu führt, dass das Polypeptid mit einer angegebenen Identität die gleiche biologische Aktivität besitzt wie das erste Polypeptid. Die Identität der Nucleotidsequenz bezieht sich auf zu einer ersten Nucleotidsequenz homologes Gen. Homolog bedeutet in Bezug auf die Nucleotidsequenz, dass das Gen allelisch sein kann. Homolog bedeutet ferner, dass das Gen aus einer anderen Spezies stammen kann, das von diesem Gen kodierte Polypeptid aber die gleiche biologische Aktivität besitzt wie das von der ersten Nucleotidsequenz kodierte Polypeptid.

Gegenstand der Erfindung sind Polypeptide mit Lipase/Acyltransferase Aktivität mit einer Aminosäuresequenz, welche zu der in SEQ ID NO. 2 angegebenen Aminosäuresequenz wenigstens 80 %, bevorzugt mindestens 98 %, besonders bevorzugt 99,8 % und insbesondere 100 % Identität besitzen. Eine Identität zu der in SEQ ID NO. 2 angegebenen Aminosäuresequenz von mindestens 80 % vorzugsweise, 96 % gilt besonders für den Teilbereich, welchen den Aminosäuren in den Positionen 190 bis 390 entspricht. Ein Polypeptid welches zu der in SEQ ID NO. 2 angegebenen Aminosäuresequenz 100 % Identität besitzt, enthält 465 Aminosäuren. Besonders bevorzugt ist eine 96 % Identität für die Teilbereiche in den Positionen 190-200, 220 bis 290 und 330 bis 385, insbesondere für die Positionen 196, 240 und 381.

Die Aminosäuresequenz des erfindungsgemäßen Enzyms wird im Sequenzprotokoll unter der Bezeichnung SEQ ID NO. 2 angegeben. Die Nukloetidsequenz dieses Enzyms wird im Sequenzprotokoll unter der Bezeichnung SEQ ID NO. 1 angegeben. Sie steht somit für Weiterentwicklungen über an sich bekannte molekularbiologische Methoden zur Verfügung.

Vergleichbare Polypeptide mit Lipase/Acyltransferase Aktivität stellen ebenfalls bevorzugte Ausführungsformen der vorliegenden Erfindung dar und werden insofern beansprucht, wie sie Aminosäure- und/oder Nucleinsäure-Sequenzen aufweisen, die innerhalb des Ähnlichkeitsbereiches zu den in SEQ ID NO. 1 und/oder SEQ ID NO. 2 angegebenen Sequenzen liegen. Dieser Ähnlichkeitsbereich umfasst alle Polypeptide, deren Aminosäuresequenz zu der in SEQ ID NO. 2 angegebenen Aminosäuresequenz mindestens, zu 80 %, zu 96 %, zu 96,5 %, zu 97 %, zu 97,5 %, zu 98 %, zu 98,5 %, zu 99 %, zu 99,5 %, zu 99,8 % oder zu 100 % identisch ist. Dies gilt insbesondere für jene Teilbereiche des Proteins, die die Aminosäuren 190 bis 390 betreffen.

Ein Polypeptid welches zu der in SEQ ID NO. 2 angegebenen Aminosäuresequenz mindestens 80 % Identität besitzt, hat eine molekulare Masse zwischen 49 und 55 kD nach Deglycosylierung, insbesondere von 54 kD. Das pH-Optimum für die katalytische Reaktion der Transesterifizierung, Hydrolyse oder Veresterung welches bei 28 °C bestimmt wurde, liegt zwischen 3 und 8,5, bevorzugt zwischen 4 und 8, insbesondere zwischen 6 und 7,5.

Ein optimaler Temperaturbereich bei der Katalyse der Hydrolyse, bestimmt beim pH-Optimum, liegt zwischen 30 und 50 °C, bevorzugt zwischen 35 und 40 °C. Ein optimaler Temperaturbereich für die Katalyse der Transesterifizierung und die Veresterung, bestimmt beim pH-Optimum, liegt zwischen 20 bis 50 °C, bevorzugt zwischen 20 und 30 °C.

Zu den erfindungsgemäßen Polypeptiden gehören auch solche Enzyme, die zu diesen hinreichende Ähnlichkeit aufweisen oder mit an sich bekannten Methoden abgeleitet werden können.

In einer besonderen Ausführungsform der Erfindung liegen die Polypeptide mit Lipase/Acyltransferase Aktivität mit einer Aminosäuresequenz, welche zu der in SEQ ID NO. 2 angegebenen Aminosäuresequenz wenigstens 80 %, bevorzugt mindestens 98 % besonders bevorzugt 99,8 % und insbesondere 100 % Identität besitzen glycosyliert vor. Die Positionen an denen das Polypeptid glycosyliert vorliegt und der Grad der Glycosylierung sind abhängig vom Organismus, der dieses Polypeptid produziert. Bevorzugt ist ein Glycosylierungsgrad von 1 bis 2 Zuckerresten pro Molekül Polypeptid.

In einer weiteren Ausführungsform der Erfindung sind die Polypeptide mit einem weiteren Peptid verknüpft. Bei einem solchen Peptid kann es sich um einen Marker handeln, der beispielsweise dazu führen kann, das gewünschte Polypeptid in der Chromatographie insbesondere der Affinitätschromatographie effektiver aufreinigen zu können. Erfindungsgemäß handelt es sich bei dem weiteren Polypeptid um den Marker his-tag. Bei his-tag handelt es sich um ein Peptid welches aus sechs Monomeren Histidin-Einheiten aufgebaut ist.

Eine besondere Ausführungsform der Erfindung sind Polypeptide mit Lipase/Acyltransferase Aktivität mit einer Aminosäuresequenz, welche zu der in SEQ ID NO. 4 angegebenen Aminosäuresequenz wenigstens 80 %, bevorzugt mindestens 98 %, besonders bevorzugt 99,8 % und insbesondere 100 % Identität besitzen.

Eine weitere bevorzugte Ausführungsform der Erfindung sind Polypeptidfragmente oder durch Deletionsmutation erhältliche Polypeptide, mit einer Lipase/Acyltransferase-Aktivität gemäß der oben beschriebenen Polypeptide.

Unter **Fragmenten** werden alle Proteine oder Peptide verstanden, die kleiner sind als natürliche Proteine, die kleiner sind als jene Proteine, die denen von SEQ ID NO. 1 oder SEQ ID NO. 2 oder SEQ ID NO. 3 oder SEQ ID NO. 4 entsprechen, zu ihnen in den entsprechenden Teilsequenzen aber hinreichend homolog sind, oder solche, die vollständig translatierten Genen entsprechen, und beispielsweise auch synthetisch erhalten werden können. Aufgrund ihrer Aminosäuresequenzen können sie den betreffenden vollständigen Proteinen zugeordnet werden. Sie können beispielsweise gleiche Strukturen annehmen oder proteolytische Aktivitäten oder Teilaktivitäten ausüben, wie beispielsweise die Komplexierung eines Substrats. Fragmente und Deletionsvarianten von Ausgangsproteinen sind prinzipiell gleichartig; während Fragmente eher kleinere Bruchstücke darstellen, fehlen den Deletionsmutanten eher nur kurze Bereiche, und damit nur einzelne Teilfunktionen.

Bei den Fragmenten kann es sich beispielsweise um einzelne Domänen handeln oder um Bruchstücke, die nicht mit den Domänen übereinstimmen. Solche Fragmente können kostengünstiger herzustellen sein, bestimmte eventuell nachteilige Charakteristika des Ausgangsmoleküls nicht mehr besitzen, wie möglicherweise einen aktivitätssenkenden Regulationsmechanismus, oder ein günstigeres Aktivitätsprofil entfalten, Derartige Proteinfragmente können auch nicht-biosynthetisch, sondern beispielsweise chemisch hergestellt werden. Die chemische Synthese kann beispielsweise dann vorteilhaft sein, wenn im Anschluss an die Synthese chemische Modifikationen vorgenommen werden sollen.

Den Fragmenten sind wegen ihrer prinzipiellen Gleichartigkeit auch durch Deletionsmutation erhältliche Polypeptide zuzuordnen. Solche können biochemisch weitgehend mit den Ausgangsmolekülen übereinstimmen oder einzelne Funktionen gerade nicht mehr aufweisen. Dies erscheint beispielsweise bei der Deletion inhibierender Bereiche besonders sinnvoll. Im Ergebnis können mit den Deletionen sowohl eine Spezialisierung als auch eine Erweiterung des Anwendungsbereichs des Proteins einhergehen. Sofern dadurch eine im weitesten Sinne Lipase/Acyltransferase Aktivität aufrechterhalten, modifiziert, spezifiziert oder auch erst erreicht wird, handelt es sich bei den durch Deletionsvarianten wie bei den Fragmenten um erfindungsgemäße Proteine; einzige zusätzliche Voraussetzung dafür ist, daß sie über die noch vorhandene homologe Teilsequenz hinweg innerhalb des angegebenen Ähnlichkeitsbereichs zu den Sequenzen SEQ ID NO. 1 und SEQ ID NO. 2 und SEQ ID NO. 3 und SEQ ID NO. 4 liegen.

Inversionsmutagenese, also eine partielle Sequenzumkehrung, kann als Sonderform der Deletion, angesehen werden. Dasselbe gilt für eine von der ursprünglichen Aminosäureabfolge abweichende Neugruppierung verschiedener Molekülteile. Sie kann als Deletionsvariante, des ursprünglichen Proteins angesehen werden.

Eine weitere Ausführungsform der Erfindung sind Derivate eines Polypeptides mit Lipase/Acyltransferase Aktivität gemäß einem der oben beschriebenen Polypeptide.

Unter **Derivaten** werden im Sinne der vorliegenden Anmeldung solche Polypeptide verstanden, deren reine Aminosäurekette chemisch modifiziert worden ist. Solche Derivatisierungen können beispielsweise biologisch im Zusammenhang mit der Proteinbiosynthese durch den Wirtsorganismus erfolgen. Hierfür können molekularbiologische Methoden eingesetzt werden. Sie können aber auch chemisch durchgeführt werden, etwa durch die chemische Umwandlung einer Seitenkette einer Aminosäure oder durch kovalente Bindung einer anderen Verbindung an das Protein. Bei solch einer Verbindung kann es sich beispielsweise um andere Proteine handeln, die beispielsweise über bifunktionelle chemische Verbindungen an erfindungsgemäße Polypeptide gebunden werden. Ebenso ist unter Derivatisierung die kovalente Bindung an einen makromolekularen Träger zu verstehen. Derartige Modifikationen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Eine weitere Ausführungsform sind deshalb solche Derivate, die durch kovalente Bindung an einen makromolekularen Träger, wie beispielsweise Polyethylenglykol oder ein Polysaccharid erhalten worden sind.

Im Sinne der vorliegenden Erfindung werden alle Polypeptide, Enzyme, Proteine, Fragmente und Derivate, sofern sie nicht explizit als solche angesprochen zu werden brauchen, unter dem Oberbegriff Polypeptide zusammengefasst.

Die enzymatische Aktivität kann durch andere Bereiche des Polypeptides, die nicht an der eigentlichen Reaktion beteiligt sind, qualitativ oder quantitativ modifiziert werden. Dies betrifft beispielsweise die Enzymstabilität, die Aktivität, die Reaktionsbedingungen oder die Substratspezifität. Denn einerseits ist nicht genau bekannt, welche Aminosäurereste des erfindungsgemäßen Polypeptide tatsächlich die Hydrolyse, Transesterifizierung und Veresterung katalysieren, und andererseits können nicht von vornherein bestimmte Einzelfunktionen definitiv von der Beteiligung an der Katalyse ausgenommen werden. Zu den Hilfsfunktionen oder Teilaktivitäten gehören beispielsweise die Bindung eines Substrats, eines Zwischen- oder Endprodukts, die Aktivierung oder die Inhibierung oder Vermittlung eines regulierenden Einflusses auf die hydrolytische Aktivität. Dabei kann es sich beispielsweise auch um die Ausbildung eines Strukturelements handeln, das fern vom aktiven Zentrum liegt, oder um ein Signalpeptid, dessen Funktion die Ausschleusung des gebildeten Proteins aus der Zelle und/oder dessen korrekte Faltung betrifft und ohne das *in vivo* in der Regel kein funktionsfähiges Enzym gebildet wird. Allerdings muß sich insgesamt eine Hydrolyse, Transesterifizierung und Veresterung katalysiert werden.

Erfindungsgemäße Polypeptide, die aus natürlichen Quellen stammen, sind bevorzugte Ausführungsformen der vorliegenden Erfindung, insbesondere wenn sie aus Mikroorganismen wie einzellige Pilze oder Bakterien stammen. Denn diese lassen sich zumeist einfacher handhaben als vielzellige Organismen oder die von Vielzellern abgeleiteten Zellkulturen. Diese stellen für spezielle Ausführungsformen sinnvolle Optionen dar.

Besonders bevorzugt sind erfindungsgemäße Polypeptide oder Derivate aus eukaryontischen Pilzen, insbesondere diese, die sekretierte Proteine unmittelbar ins umgebende Medium abgeben können.

Ganz besonders bevorzugt sind erfindungsgemäße Polypeptide oder Derivate die erhältlich sind aus Mikroorganismen, die ausgewählt sind aus der Gruppe, die gebildet wird von *Candida parapsilosis* und bevorzugt *Candida parapsilosis CBS 604, Candida antarctica (Trychosporon oryzae, Pseudozyma antarctica) Candida glabrata, Candida albicans, Candida maltosa, Candida tropicalis, Candida viswanathii, Issatchenkia orientalis (Candida krusei), Kluyveromyces marxianus (C. kefyr, C. pseudotropicalis), Pichia guilliermondii (Candida guilliermondii), Geotrichum candidum, Fusarium solani* und *Aeromonas aerophila*.

Unter den erfindungsgemäßen Polypeptiden oder Derivate aus Candida-Species, wiederum, sind die aus *Candida parapsilosis* bevorzugt, darunter insbesondere aus *Candida parapsilosis CBS 604,* denn aus diesem wurde die Ausführungsform des erfindungsgemäßen Enzyms, deren zugehörige Sequenzen im Sequenzprotokoll angegeben sind, ursprünglich erhalten.

Aus produktionstechnischen Gründen sind jeweils solche Stämme bevorzugt, die das gebildete Polypeptid in das sie umgebende Medium abgeben.

Ein weiterer Gegenstand der Erfindung sind Nucleinsäuren, die für ein Polypeptid mit Lipase/Acyltransferase Aktivität codieren, deren Nucleotidsequenz zu der in SEQ ID NO. 1 angegebenen Nucleotidsequenz zu 100 % identisch ist, insbesondere über den Teilbereich, der den Aminosäuren 190 bis 390 gemäß der SEQ ID NO. 2 entspricht. Weiterhin sind Nucleinsäuren beansprucht, kodierend für eine Aminosäuresequenz, welche zu der in SEQ ID NO. 2 angegebenen Aminosäuresequenz wenigstens 80 %, besonders 99,8 % und insbesondere 100 % Identität besitzt, wobei die Aminosaüresequenz ein Polypeptid mit Lipase/Acyl transferase Activität darstellt. Bevorzugt sind Nucleinsäuren, kodierend für eine Aminosäuresequenzen, welche zu der in SEQ ID NO. 2 angegebenen Aminosäuresequenz in den Positionen 190 bis 390 mindestens zu 96 % identisch sind, wobei die Aminosaüresequenz ein Polypeptid mit Lipase/Acyltransferase Activität darstellt. Insbesondere bevorzugt sind Nucleinsäuren, die für eines der oben beschriebenen Polypeptide oder Derivate codieren. Der Ähnlichkeitsbereich umfasst auch alle Polypeptide, deren Nucleotidsequenz zu der in SEQ ID NO. 1 angegebenen Nucleotidsequenz mindestens zu 85 %, zu 87,5 %, zu 90 %, zu 92,5 %, zu 95 %, zu 96 %, zu 97 %, zu 98 %, zu 99 % oder zu 100 % identisch ist.

Ein weiterer Gegenstand der Erfindung sind Nucleinsäuren die für ein Polypeptid mit Lipase/Acyltransferase Aktivität kodieren, deren Nucleotidsequenz zu der in SEQ ID NO. 3 angegebenen Nucleotidsequenz identisch ist.

In den Schutzbereich mit einbezogen sind Nucleinsäuren kodierend für eine Aminosäuresequenz, welche zu der in SEQ ID NO. 4 angegebenen Aminosäuresequenz wenigstens 80 % Identität besitzt, wobei die Aminosaüresequenz ein Polypeptid mit Lipase/Acyltransferase Activität darstellt. Der Ähnlichkeitsbereich umfasst auch alle Polypeptide, deren Nucleotidsequenz zu der in SEQ ID NO. 3 angegebenen Nucleotidsequenz mindestens zu 85 %, zu 87,5 %, zu 90 %, zu 92,5 %, zu 95 %, zu 96 %, zu 97 %, zu 98 %, zu 99 % oder zu 100 % identisch ist.

Unter Nucleinsäuren sind im Sinne der vorliegenden Anmeldung die natürlicherweise aus Nucleotiden aufgebauten als Informationsträger dienenden Moleküle zu verstehen, die für die lineare Aminosäureabfolge in Proteinen oder Enzymen codieren. Sie können als Einzelstrang, als ein zu diesem Einzelstrang komplementärer Einzelstrang oder als Doppelstrang vorliegen. Als der natürlicherweise dauerhaftere Informationsträger ist die Nucleinsäure DNA für molekularbiologische Arbeiten bevorzugt. Demgegenüber wird für die Realisierung der Erfindung in natürlicher Umgebung, wie beispielsweise in einer exprimierenden Zelle, eine RNA gebildet, weshalb erfindungswesentliche RNA-Moleküle ebenfalls Ausführungsformen der vorliegenden Erfindung darstellen.

Bei DNA sind die Sequenzen beider komplementärer Stränge in jeweils allen drei möglichen Leserastern zu berücksichtigen. Ferner ist zu berücksichtigen, dass verschiedene Codon-Triplets für dieselben Aminosäuren codieren können, so dass eine bestimmte Aminosäure-Abfolge von mehreren unterschiedlichen und möglicherweise nur geringe Identität aufweisenden Nucleotidsequenzen abgeleitet werden kann (Degeneriertheit des genetischen Codes). Außerdem weisen verschiedene Organismen Unterschiede im Gebrauch dieser Codons auf. Aus diesen Gründen müssen sowohl Aminosäuresequenzen als auch Nukleotidsequenzen in die Betrachtung des Schutzbereichs einbezogen werden und angegebene Nukleotidsequenzen sind jeweils nur als eine beispielhafte Codierung für eine bestimmte Aminosäurefolge anzusehen.

Die einem Protein entsprechende Informationseinheit wird auch im Sinne der vorliegenden Anmeldung als Gen bezeichnet.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind beispielsweise aus dem "Lexikon der Biochemie", Spektrum Akademischer Verlag, Berlin, 1999, Band 1, S. 267-271 und Band 2, S. 227-229, bekannt.

Änderungen der Nukleotidsequenz, wie sie beispielsweise durch an sich bekannte molekularbiologische Methoden herbeigeführt werden können, werden als Mutationen bezeichnet. Je nach Art der Änderung kennt man beispielsweise Deletions-, Insertions- oder Substitutionsmutationen oder solche, bei denen verschiedene Gene oder Teile von Genen miteinander fusioniert (shuffling) werden; dies sind Genmutationen. Die zugehörigen Organismen werden als Mutanten bezeichnet. Die von mutierten Nukleinsäuren abgeleiteten Proteine werden als Varianten bezeichnet. So führen beispielsweise Deletions-, Insertions- Substitutionsmutationen oder Fusionen zu deletions-, insertionssubstitutionsmutierten oder Fusionsgenen und auf Proteinebene zu entsprechenden Deletions-, Insertions- oder Substitutionsvarianten, beziehungsweise Fusionsproteinen.

Eine weitere Lösung der erfindungsgemäßen Aufgabe stellen die Organismen dar, die ein erfindungsgemäßes Protein oder Derivat natürlicherweise bilden oder Nukleinsäuren enthalten, die für ein erfindungsgemäßes Polypeptide oder Derivate kodieren. Denn deren Auffinden ermöglicht das Umsetzen des Erfindungsgedankens. Derartige Organismen sind in Anwendung allgemein bekannter Techniken, beispielsweise durch Isolieren von Stämmen aus einem natürlichen Habitat oder durch Screening von Genbanken erhältlich. Die in SEQ ID NO. 1 angegebene Nukleotidsequenz kann hierbei beispielsweise als Sonde zum Screening eingesetzt werden oder als Vorlage für die Konstruktion entsprechender PCR-Primer dienen. Analog dazu können kurzkettige oder vollständige Peptide mit Aminosäuresequenzen nach SEQ ID NO, 2 zur Bildung entsprechender Antiseren verwendet werden, mit deren Hilfe entsprechende Organismen, beziehungsweise die von ihnen freigesetzten Proteine identifiziert werden können.

Entsprechend der oben gemachten Ausführungen sind Mikroorganismen, weil diese vor allem aufgrund der Kultivierbarkeit und als Produktionsorganismen mit einer besonders hohen Produktionsleistung in technischen Prozessen etabliert sind, vorzugsweise Hefe-Pilze, hierunter solcher der Gattung Candida, insbesondere *Candida parapsilosis* und ganz besonders um *Candida parapsilosis CBS 604* bevorzugt.

Aus produktionstechnischen Gründen sind jeweils solche Stämme bevorzugt, die das gebildete Polypeptid in das sie umgebende Medium abgeben.

Es ist möglich, daß natürlich vorkommende Produzenten zwar ein erfindungsgemäßes Enzym herstellen können, dieses aber unter den zunächst ermittelten Bedingungen nur in geringem Maße exprimieren und/oder in das umgebende Medium abgeben. Sie fallen dennoch solange unter den Schutzbereich der vorliegenden Erfindung, wie die Möglichkeit besteht, geeignete Umweltbedingungen oder niedermolekulare oder sonstige Faktoren experimentell zu ermitteln, unter deren Einwirken sie zu einer Produktion des erfindungsgemäßen Proteins angeregt werden können, die eine wirtschaftliche Nutzung sinnvoll erscheinen lässt. Solch ein Regulationsmechanismus kann für die biotechnologische Produktion gezielt eingesetzt werden, zum Beispiel zur Regulation der verantwortlichen Promotoren.

Je nach Gewinnung, Aufarbeitung oder Präparation eines Proteins kann es mit diversen anderen Stoffen vergesellschaftet sein, insbesondere wenn es aus natürlichen Produzenten dieses Proteins gewonnen worden ist. Es kann dann, aber auch unabhängig davon, mit bestimmten anderen Stoffen gezielt versetzt worden sein, beispielsweise zur Erhöhung seiner Lagerstabilität. Unter dem Begriff des erfindungsgemäßen Proteins sind deshalb zusätzlich auch alle Präparationen des eigentlichen erfindungswesentlichen Proteins zu verstehen. Das ist auch unabhängig davon, ob es in einer bestimmten Präparation tatsächlich diese enzymatische Aktivität entfaltet oder nicht. Denn es kann gewünscht sein, dass es bei der Lagerung keine oder nur geringe Aktivität besitzt, und erst zum Zeitpunkt der Verwendung seine Funktion entfaltet. Dies kann beispielsweise vom Faltungszustand des Proteins abhängig sein oder aus der reversiblen Bindung eines oder mehrere Begleitstoffe aus der Präparation oder aus einem sonstigen Kontrollmechanismus resultieren.

Nucleinsäuren bilden den Ausgangspunkt für molekularbiologische Untersuchungen und Weiterentwicklungen. Solche Methoden sind beispielsweise in dem Handbuch von Fritsch, Sambrook und Maniatis "Molecular cloning: a laboratory manual", Cold Spring Harbour Laboratory Press, New York, 1989 beschrieben. Auf dem Gen, insbesondere auf dem klonierten Gen beruhen auch alle im Stand der Technik unter dem Begriff Protein Engineering zusammengefassten gentechnischen und Protein-biochemischen Methoden. Mit solchen können erfindungsgemäße Polypeptide in Hinblick auf verschiedene Verwendungen weiter optimiert werden, beispielsweise durch Punktmutagenese oder durch Fusion mit Sequenzen aus anderen Genen

Einem eigenen Erfindungsgegenstand werden Vektoren zugerechnet, die einen der beschriebenen Nucleinsäurebereiche enthalten die für ein erfindungsgemäßes Polypeptid mit Lipase/Acyltransferase Aktivität codieren.

Denn um mit Nukleinsäuren umzugehen, wird die DNA geeigneterweise in einen Vektor kloniert. Vektoren sind DNA-Moleküle, die sich als Transprotmoleküle (Vehikel) zur Einschleusung (Transformation) von Fremd-DNA in Wirtszellen eignen und dort evt. autonom replizierbar sind. Häufig verwendeten Vektoren sind Plasmide, d.h. extrachromosomale, ringförmige, doppelsträngige Bakterien-DNA, die sich durch geeignete Methoden in andere Mikroorganismen einbringen lässt und dort vermehrbar ist.

Zu den Vektoren gehören beispielsweise solche, die sich von bakteriellen Plasmiden, von Viren oder von Bacteriophagen ableiten, oder überwiegend synthetische Vektoren oder Plasmide mit Elementen verschiedenster Herkunft. Mit den weiteren jeweils vorhandenen genetischen Elementen vermögen Vektoren, sich in den betreffenden Wirtszellen über mehrere Generationen hinweg als stabile Einheiten zu etablieren. Es ist dabei im Sinne der Erfindung unerheblich, ob sie sich extrachomosomal als eigene Einheiten etablieren oder in ein Chromosom integrieren. Welches der zahlreichen aus dem Stand der Technik bekannten Systeme gewählt wird, hängt vom Einzelfall ab. Ausschlaggebend können beispielsweise die erreichbare Kopienzahl, die zur Verfügung stehenden Selektionssysteme, darunter vor allem Antibiotikaresistenzen, oder die Kultivierbarkeit der zur Aufnahme der Vektoren befähigten Wirtszellen sein.

Die Vektoren bilden geeignete Ausgangspunkte für molekularbiologische und biochemische Untersuchungen des betreffenden Gens oder zugehörigen Proteins und für erfindungsgemäße Weiterentwicklungen und letztlich für die Amplifikation und Produktion erfindungsgemäßer Proteine. Sie stellen insofern Ausführungsformen der vorliegenden Erfindung dar, als die Sequenzen der enthaltenen erfindungsgemäßen Nukleinsäurebereiche innerhalb des oben näher bezeichneten Homologiebereichs liegen.

Bevorzugte Ausführungsformen der vorliegenden Erfindung sind Klonierungsvektoren. Diese eignen sich neben der Lagerung, der biologischen Amplifikation oder der Selektion des interessierenden Gens für die Charakterisierung des betreffenden Gens, etwa über das Erstellen einer Restriktionskarte oder die Sequenzierung. Klonierungsvektoren sind auch deshalb bevorzugte Ausführungsformen der vorliegenden Erfindung, weil sie eine transportierbare und lagerfähige Form der beanspruchten DNA darstellen. Sie sind auch bevorzugte Ausgangspunkte für molekularbiologische Techniken, die nicht an Zellen gebunden sind, wie beispielsweise die Polymerasekettenreaktion.

Expressionsvektoren besitzen Teilsequenzen, die sie dazu befähigen, in den für die Produktion von Proteinen optimierten Wirtsorganismen zu replizieren und dort das enthaltene Gen zur Expression zu bringen. Bevorzugte Ausführungsformen sind Expressionsvektoren, die selbst die zur Expression notwendigen genetischen Elemente tragen. Die Expression wird beispielsweise von Promotoren beeinflusst, welche die Transkription des Gens regulieren. So kann die Expression durch den natürlichen, ursprünglich vor diesem Gen lokalisierten Promotor erfolgen, aber auch nach gentechnischer Fusion sowohl durch einen auf dem Expressionsvektor bereitgestellten Promotor der Wirtszelle als auch durch einen modifizierten oder einen völlig anderen Promotor eines anderen Organismus.

Bevorzugte Ausführungsformen sind solche Expressionsvektoren, die über Änderungen der Kulturbedingungen oder Zugabe von bestimmten Verbindungen, wie beispielsweise die Zelldichte oder spezielle Faktoren, regulierbar sind. Expressionsvektoren ermöglichen, dass das zugehörige Protein heterolog, also in einem anderen Organismus als dem, aus dem es natürlicherweise gewonnen werden kann, produziert wird. Auch eine homologe Proteingewinnung aus einem das Gen natürlicherweise exprimierenden Wirtsorganismus über einen passenden Vektor liegt innerhalb des Schutzbereichs der vorliegenden Erfindung. Diese kann den Vorteil aufweisen, dass natürliche, mit der Translation in einem Zusammenhang stehende Modifikationsreaktionen an dem entstehenden Protein genauso durchgeführt werden, wie sie auch natürlicherweise ablaufen würden.

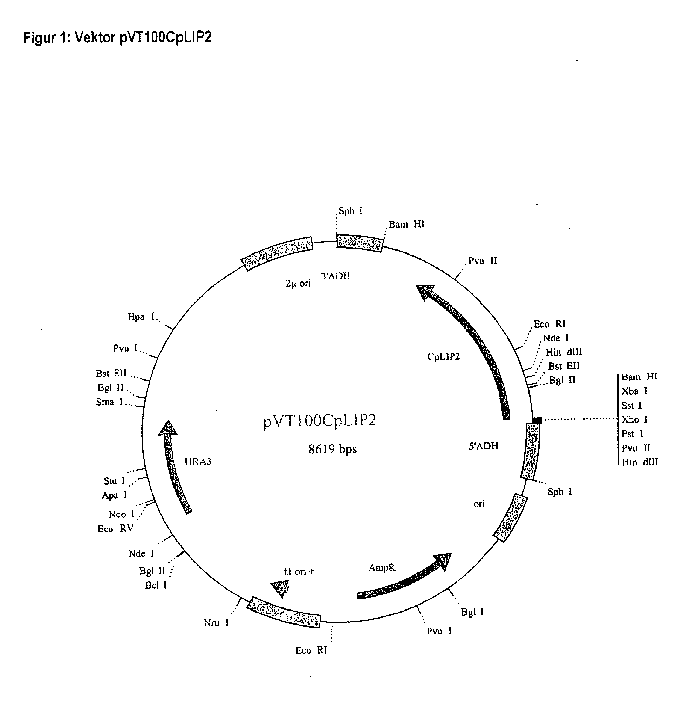

Zur Gewinnung der erfindungsgemäßen Polypeptide werden Mikroorganismen kultiviert, die mit einem Expressionsvektor transformiert wurden, der das für das entsprechende Enzym kodierende Strukturgen enthält. Die Expressionsvektoren wurden hierbei durch weiter unten beschriebene Verfahren gewonnen. Die besonders bevorzugten Mikroorganismen die mit dem Expressionsvektor transformiert werden, sind *Saccharomyces cerevisiae* und *Pichi pastoris*. Bevorzugte Vektoren sind Plasmide deren Restriktionskarten in den **Figuren 1 bis 3** abgebildet sind.

Zu den im Rahmen der Erfindung verwendeten Vektoren zählen die, die durch Schneiden mit geeigneten Restriktionsendonukleasen, bevorzugt *Bam*HI oder *Sna*BI und nachfolgende Rekombination mit den entsprechenden N- bzw. C-terminalen Hälften des Enzymstrukturgens gebildet werden. Restriktionsendonucleasen sind Enzyme, die substratspezifisch doppelsträngige DNA dadurch in Fragmente zerlegen, dass sie Phosphatdiesterbindungen zwischen einzelnen Nucleotidbausteinen der DNA spalten. Alle Restriktionsendonucleasen vermögen bestimmte Basensequenzen der DNA zu erkennen, welche für die Aktivität der betreffenden Restriktionsendonucleasen spezifische Wirkungsorte (Schnittstellen) markieren. Beim Schneiden (Restriktion) doppelsträngiger DNA entstehen bei einigen Restriktionsendonucleasen spezifische sogenannte "überstehende Enden", die unter bestimmten Renaturierungsbedingungen wieder miteinander oder mit entsprechenden (komplementären) überstehenden Enden anderweitig gewonnener DNA-Fragmente verbunden (ligiert) werden können (Rekombination).

Ausführungsformen der vorliegenden Erfindung können auch zellfreie Expressionssysteme sein, bei denen die Proteinbiosynthese *in vitro* nachvollzogen wird. Derartige Expressionssysteme sind im Stand der Technik ebenfalls etabliert.

Eine weitere Ausführungsform dieses Erfindungsgegenstands stellen Zellen dar, die einen der oben definierten Vektoren, insbesondere einen Klonierungs- oder einen Expressionsvektor enthalten. Denn insbesondere im Zuge molekularbiologischer Arbeiten, wie sie beispielsweise für die Mutagenese, die Sequenzierung oder Lagerung der Vektoren nötig sind, erfolgt deren Transformation in entsprechende Zellen. Hierfür können, abhängig von der Methode, beispielsweise gram-positive, insbesondere aber auch gram-negative Bakterien geeignet sein.

Eine weitere Ausführungsform sind Wirtszellen, die ein Polypeptid oder Derivat des ersten Erfindungsgegenstands exprimieren oder zu deren Expression angeregt werden können, vorzugsweise unter Einsatz eines oben definierten Expressionsvektors,

Denn die bevorzugte *In-vivo*-Synthese eines erfindungsgemäßen Polypeptides erfordert den Transfer des zugehörigen Gens in eine Wirtszelle. Als Wirtszellen eignen sich prinzipiell alle Organismen, das heißt Prokaryonten, Eukaryonten oder Cyanophyta. Bevorzugt sind solche Wirtszellen, die sich genetisch gut handhaben lassen, was beispielsweise die Transformation mit dem Expressionsvektor und dessen stabile Etablierung angeht, beispielsweise einzellige Pilze oder Bakterien. Zudem zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Häufig müssen aus der Fülle an verschiedenen nach dem Stand der Technik zur Verfügung stehenden Systeme die optimalen Expressionssysteme für den Einzelfall experimentell ermitteln werden. Jedes erfindungsgemäße Protein kann auf diese Weise aus einer Vielzahl von Wirtsorganismen gewonnen werden.

Bevorzugte Ausführungsformen stellen solche Wirtszellen dar, die aufgrund genetischer Regulationselemente, die beispielsweise auf dem Expressionsvektor zur Verfügung gestellt werden, aber auch von vornherein in diesen Zellen vorhanden sein können, in ihrer Aktivität regulierbar sind. Beispielsweise können diese zur Expression angeregt werden durch kontrollierte Zugabe von chemischen Verbindungen wie beispielsweise Methanol, die als Aktivatoren dienen, durch Änderung der Kultivierungsbedingungen oder bei Erreichen einer bestimmten Zelldichte, Dies ermöglicht eine sehr wirtschaftliche Produktion der interessierenden Proteine.

Eine Variante dieses Versuchsprinzips stellen Expressionssysteme dar, bei denen zusätzliche Gene, beispielsweise solche, die auf anderen Vektoren zur Verfügung gestellt werden, die Produktion erfindungsgemäßer Proteine beeinflussen. Hierbei kann es sich um modifizierende Genprodukte handeln oder um solche, die mit dem erfindungsgemäßen Protein gemeinsam aufgereinigt werden sollen, etwa um dessen Funktion zu beeinflussen. Dabei kann es sich beispielsweise um andere Proteine oder Enzyme, um Inhibitoren oder um solche Elemente handeln, die die Wechselwirkung mit verschiedenen Substraten beeinflussen.

Bevorzugte Wirtszellen sind prokaryontische oder bakterielle Zellen. Bakterien zeichnen sich gegenüber Eukaryonten in der Regel durch kürzere Generationszeiten und geringere Ansprüche an die Kultivierungsbedingungen aus. Dadurch können kostengünstige Verfahren zur Gewinnung erfindungsgemäßer Proteine etabliert werden.

Insbesondere bevorzugt sind Wirtszellen, insbesondere Bakterien, die das gebildete Protein oder Derivat ins umgebende Medium sekretieren, so dass die exprimierten erfindungsgemäßen Proteine direkt aufgereinigt werden können.

Bevorzugt ist die heterologe Expression. Grampositive Bakterien, wie beispielsweise Actinomycten oder Bacilli, besitzen keine äußere Membran, so dass sie sekretierte Proteine unmittelbar in das sie umgebende Medium abgeben. Zu den für die heterologe Expression bevorzugten Bakterien gehören somit solche der Gattung Bacillus, insbesondere solche der Species, die unten aufgelistet sind.

Auch gram-negative Bakterien können für die heterologe Expression genutzt werden. Bei ihnen werden eine Vielzahl von Proteinen in den periplasmatischen Raum sekretiert, also in das Kompartiment zwischen den beiden die Zellen einschließenden Membranen. Dies kann für spezielle Anwendungen vorteilhaft sein. Hierzu gehören beispielsweise solche der Gattungen Klebsiella oder Escherichia, bevorzugt der Spezies die unten mitaufgelistet sind.

Auch eukaryontische Zellen können sich zur Produktion erfindungsgemäßer Polypeptide eignen. Beispiele dafür sind Hefen wie *Saccharomyces* oder *Kluyveromyces*. Dies kann beispielsweise dann besonders vorteilhaft sein, wenn die Proteine im Zusammenhang mit ihrer Synthese spezifische Modifikationen erfahren sollen, die derartige Systeme ermöglichen. Dazu gehören beispielsweise die Bindung niedermolekularer Verbindungen wie Membrananker oder Oligosaccaride.

Besonders bevorzugt für die Produktion erfindungsgemäßer Polypeptide aus transformierten Wirtszellen sind Mikroorganismen, die ausgewählt sind aus der Gruppe, die gebildet wird von *Candida parapsilosis, Saccharomyces cerevisiae, Schizosaccharomyces pombe, Pichia pastoris, Pichia boidinii, Pichia stipitis, Hansenula polymorpha, Kluyveromyces lactis, Schwanniomyces castellii, Yarrowia lipolytica, Escherishia coli, Bacillus subtilis, Bacillus amylolichefaciens, Bacillus stearothermophilus, Bacillus licheniformis, Lactococcus lactis, Streptococcus lactis, Lactobacillus bulgaricus, Aspergillus orizae, Aspergillus niger, Trichoderma reesei, Mucor sp* und *Rhizopus sp.*

Die transformierten Wirtszellen oder auch Transformanten genannt, werden anschließend in an sich bekannter Weise, bevorzugt wie in den Beispielen beschrieben, kultiviert und die gebildeten erfindungsgemäßen Polypeptide isoliert.

Alle bereits oben ausgeführten Elemente können zu Verfahren kombiniert werden, um erfindungsgemäße Polypeptide herzustellen. Diese Verfahren stellen daher einen weiteren Gegenstand der Erfindung dar. Es ist dabei für jedes erfindungsgemäße Protein eine Vielzahl von Kombinationsmöglichkeiten an Verfahrensschritten denkbar. Sie alle verwirklichen die der vorliegenden Erfindung zugrundeliegende Idee, nämlich Vertreter eines über die Lipase/Acyltransferase Aktivität und gleichzeitig die hohe Homologie zu den in den Sequenzprotokollen angegebenen Sequenzen definierten Proteintyps mithilfe der zugehörigen genetischen Information quantitativ herzustellen. Das optimale Verfahren muss für jeden konkreten Einzelfall experimentell ermittelt werden.

Prinzipiell wird dabei folgendermaßen vorgegangen: Erfindungsgemäße Nukleinsäuren, also solche die innerhalb des oben definierten Ähnlichkeitsbereiches zu der Seqzuenz von SEQ ID NO. 1 oder SEQ ID NO. 3 liegen, werden geeigneterweise in Form der DNA in einen geeigneten Expressionsvektor ligiert. Dieser wird in die Wirtszelle transformiert, beispielsweise in Zellen eines leicht zu kultivierenden Bakterienstammes, der die Proteine, deren Gene unter der Kontrolle entsprechender genetischer Elemente stehen, in das umgebende Nährmedium ausschleust; regulierende Elemente dafür können beispielsweise vom Expressionsvektor zur Verfügung gestellt werden. Aus dem umgebenden Medium kann das erfindungsgemäße Protein über mehrere Aufreinigungsschritte, wie beispielsweise Fällungen oder Chromatographien, aufgereinigt werden. Ein Fachmann ist in der Lage, ein System, welches im Labormaßstab experimentell optimiert worden ist, auf einen großtechnischen Produktionsmaßstab zu übertragen.

Ein weiterer Gegenstand der Erfindung ist die Verwendung von natürlichen und/oder rekombinanten Mikroorganismen wie sie oben beschrieben wurden, enthaltend eine Nucleinsäure zur Herstellung eines erfindungsgemäßen, oben beschriebenen Polypeptides.

Weitere erfindungsgemäße Verwendung der oben beschriebenen Nucleinsäuren und/oder Aminosäuresequenz, welche zu der in SEQ ID NO. 2 und/oder zu der in SEQ ID NO. 4 angegebenen Aminosäuresequenz wenigstens 49 %, vorzugsweise 80 %, bevorzugt mindestens 98 % besonders bevorzugt 99,8 % und insbesondere 100 % Identität besitzen zum Auffinden neuer Acyltransferasen.

Dieses Auffinden neuer Enzyme wird auch als Screening bezeichnet. Speziell screent man Genbanken bestimmter Organismen nach allgemeinen Methoden, wie beispielsweise in Fritsch, Sambrook und Maniatis "Molecular cloning: a laboratory manual", Cold Spring Harbour Laboratory Press, New York, 1989 angegeben sind.

Durch Vergleich mit bekannten Enzymen, die beispielsweise in allgemein zugänglichen Datenbanken hinterlegt sind, lassen sich aus der Aminosäure- oder Nucleotidsequenz charakteristische Molekülteile wie beispielsweise Strukturelemente oder die enzymatische Aktivität eines betrachteten Enzyms folgern. Solch ein Vergleich geschieht dadurch, dass ähnliche Abfolgen in den Nukleotid- oder Aminosäuresequenzen der betrachteten Proteine einander zugeordnet werden. Dies nennt man Homologisierung. Eine tabellarische Zuordnung der betreffenden Positionen wird als Alignment bezeichnet. Bei der Analyse von Nukleotidsequenzen sind wiederum beide komplementären Stränge von jeweils allen drei möglichen Leserastern zu berücksichtigen; ebenso die Degeneriertheit des genetischen Codes und die organismenspezifische Codon-Usage. Inzwischen werden Alignments über Computerprogramme erstellt, wie beispielsweise durch die Algorithmen FASTA oder BLAST; dieses Vorgehen wird beispielsweise von D. J. Lipman und W. R. Pearson (1985) in Science, Band 227, S. 1435-1441 beschrieben. Eine Zusammenstellung aller in den verglichenen Sequenzen übereinstimmenden Positionen wird als Consensus-Sequenz bezeichnet.

Solch ein Vergleich erlaubt auch eine Aussage über die Ähnlichkeit oder Homologie der verglichenen Sequenzen zueinander. Diese wird in Prozent Identität, das heißt dem Anteil der identischen Nukleotide oder Aminosäurereste an denselben Positionen widergegeben. Ein weiter gefasster Homologiebegriff bezieht die konservierten Aminosäure-Austausche in diesen Wert mit ein. Es ist dann von Prozent Ähnlichkeit die Rede. Solche Aussagen können über ganze Proteine oder Gene oder nur über einzelne Bereiche getroffen werden.

Homologe Bereiche von verschiedenen Proteinen sind zumeist solche mit gleichen Strukturelementen und/oder Funktionen, die sich durch Übereinstimmungen in der primären Aminosäuresequenz erkennen lassen. Sie geht bis zu völligen Identitäten in kleinsten Bereichen, sogenannten Boxen, die nur wenige Aminosäuren umfassen und meist für die Gesamtaktivität essentielle Funktionen ausüben. Unter den Funktionen der homologen Bereiche sind kleinste Teilfunktionen der vom gesamten Protein ausgeübten Funktion zu verstehen, wie beispielsweise die Ausbildung einzelner Wasserstoffbrückenbindungen zur Komplexierung eines Substrats oder Übergangskomplexes.

Aufgrund von Alignments können für erfindungsgemäße Polypeptide weitgehend dieselben Sekundär- und Tertiärstrukturen angenommen werden wie für die zur Homologisierung herangezogenen Proteine. Deren Strukturelemente können in allgemein zugänglichen Datenbanken wie beispielsweise am EMBL-European Bioinformatics Institute (EBI) in Cambridge, Großbritannien (http://www.ebi.ac.uk), Swiss-Prot oder GenBank (National Center for Biotechnology Information NCBI, National Institutes of Health, Bethesda, MD, USA) abgerufen werden. Sollten sich hiervon abweichende Strukturen ergeben oder sich herausstellen, dass verschiedene Faltungsvarianten mit variierenden Eigenschaften existieren, was beispielsweise die optimalen Reaktionsbedingungen oder die Substratspezifität angeht, so werden diese alle in den Schutzbereich der vorliegenden Erfindung einbezogen. Denn zum einen kann die Faltung von den Herstellungsbedingungen abhängen, beispielsweise bei Anwesenheit oder Fehlen des Leader-Peptids. Zum anderen können sich diese Varianten für jeweils verschiedene Einsatzmöglichkeiten als besonders geeignet herausstellen.

Ein weiterer Gegenstand der Erfindung ist die Verwendung von beschriebenen Polypeptiden als Katalysatoren bei Acyltransferreaktionen, insbesondere bei Reaktionen die ausgewählt sind aus der Gruppe die gebildet wird von Alkoholyse von Estern, speziell von Glycerolen oder Sterolen, Alkoholyse von Thioestern, Thiolyse von Estern, Aminolyse eines Esters mit Hydroxylaminen oder Hydrazinen; Reaktion eines Esters mit Hydrogen-Peroxiden und enantioselektive Synthese von Estern, Thioestern oder Lactonen durch Alkoholyse. Spezielle Reaktionen die von den erfindungsgemäßen Polypeptiden katalysiert werden, sind beispielsweise beschrieben in: a) Fournand et al. J. Mol. Catalysis B, 1998, 5, 207-211; b) Briand et al. Eur. J. Biochem. 1995, 228, 169-175.

### Beispiele

### 1. Beispiel: Kultivierung des Stammes und Isolierung des Polypeptides

Der Stamm *Candida parapsilosis* (Ashford) Langeron und Talice, CBS 604 wurde beim Centraalbureau voor Schimmelcultures, Yeast devision, Delft Niederlande hinterlegt.

Die Anzucht erfolgte in der gleichen Weise wie bei Briand et al. in Eur. J. Biochem., 1995, 228, 169-175 beschrieben. Die Hauptkultur wurde mit 100 mM Phosphatpuffer auf pH 6,5 eingestellt und mit 5 g/l Glukose als C-Quelle versetzt.

Am Ende der exponentiellen Wachstumsphase wurde die Kulturbrühe zentrifugiert (7000 g für 15 min) und die Lipase/Acyltransferase aus dem flüssigen Überstand erhalten. Die Aufreinigung des Polypeptides erfolgte nach der Methode, beschrieben in Riaublanc A. et al. J. Am. Oil Chem. Soc. 1993, 70, 497-500.

### 2. Beispiel: Molekularbiologische Arbeitsschritte

Alle molekularbiologischen Arbeitsschritte folgen Standardmethoden, wie sie beispielsweise in Handbüchern wie dem von Fritsch, Sambrook und Maniatis "Molecular cloning: a laboratory manual", Cold Spring Harbour Laboratory Press, New York, 1989 angegeben sind.

Der Gehalt an Lipase/Acyltransferase wurde in units (U) gemessen, bestimmt als Gehalt an Ölsäure die pro Minute erhalten wird bei der Hydrolyse von Trioleylglycerol unter den Bedingungen beschrieben bei Briand et al. in Eur. J. Biochem. 1995; 228; 169-175. Die Proteinkonzentration wurde nach der Methode von Bradford (1976; Anal, Biochem. 72; 248-254) bestimmt.

### 3. Beispiel: Expression eines Gens enthaltend die Nucleinsäure nach SEC ID NO. 1 in Saccharomyces cerevisiae

Zur Expression der gewünschten Nucleinsäuresequenz wurde zunächst mit der Restriktionsendonuclease *Bam*HI DNA partiell hydrolysiert. Mittels degenerierter PCR wurden Primer konstruiert, welche die Nucleinsäure nach SEQ ID NO. 1 enthalten. Folgende Primer-Paare wurden verwendet (Start und Stop-Codon sind unterstrichen; BamHI-Restriktionsseite ist fett gedruckt):
forward 5'-CTC**GGATCC**ATGCGTTACTTTGCTATTGC
reverse 5'-CAC**GGATCC**TTAAAAAGCAAAACGTTCCAACTTGAGCAATCC

Für die PCR Amplifikation wurde folgendes Zeit/Temperatur-Programm durchgeführt: 5 min bei 95 °C denaturiert und danach 30 Zyklen von 1 min bei 95 °C, 1 min bei 50 °C, 1 min bei 72 °C und als letzten Schritt 10 min bei 72 °C.

Die Fragmente aus der PCR wurden mit der Restriktionsendonuclease *Bam*HI digestiert und anschliessend in den mit der Restriktionsendonuclease *Bam*HI geschnittenen Vektor pVT100-U zum Plasmid ligiert. Erhalten wurde der Vektor pVT100CpLIP2 nach **Figur 1** (replikatives Plasmid). Die Abwesenheit von Mutationen wurde durch Sequenzierung der Inserts geprüft. Die Transformation der neukombinierten DNA in den Stamm *Saccharomyces cerevisiae* W303-1a wurde durchgeführt nach der Elektroporations-Methode beschrieben von Becker et al. in Methods in Enzymology, 1991, 194, 182-187. Die Transformanten wurden auf YNB Medium ohne Uracil selektiert (6,7 g/l Yeast nitrogen base ohne Aminosäure von der Firma [Difco], 20 g/l Glukose, 150 mg/l Leucin, 100 mg/l Adenin, 100 mg/l Histidin, 100 mg/l Tryptophan) mit einer Frequenz von 1-2x10⁴ Tranformanten pro µg DNA. Die Transformanten wurden selektiert in einem Plattentest auf Lipase-Aktivität nach der Methode von Kouker beschrieben in : Kouker G. et al., Applied Environ. Microbiol. 1987, 59, 211-213.

Der selektierte Transformant wurden in einem Schüttelkolben bei 28 °C im YPD Medium kultiviert. (YPD = 10 g/l Yeast extract [Difco], 20 g/l Bacto peptone [Difco], 60 g/l Glukose, 150 mg/l Leucin, 100 mg/l Adenine, 100 mg/l Histidin und 100 mg/l Tryptophan). Die Kulturbrühe wurde nach 36 Stunden Fermentation geerntent und der Überstand der Kulturlösung durch Zentrifugieren vom Rückstand getrennt. Der Überstand enthielt 2500 U der rekombinanten Lipase/Acyltransferase pro Liter und eine spezifische Aktivität von 0,7 U/mg. Nach der Aufkonzentration durch Ultrafiltration und hydrophober Chromatographie an Phenylsepharose 6 Fast-Flow Gel konnten 10 % der Aktivität wieder erhalten werden, mit einer spezifischen Aktivität von 80 U/mg.

### 4. Beispiel: Expression eines Gens enthaltend SEQ ID NO. 1 in Pichia pastoris

Die Lipase/Acyltransferase wurde exprimiert als Fusion zu einem N-terminalem Peptid, welches für das Sekretionssignal des α-Faktors aus *Saccharomyces cerevisiae* kodiert. Zunächst wurde das Gen korrespondierend zu SEQ ID NO. 1 PCR amplifiziert und dadurch ein geschnittenes Gen des gereiften Genes erhalten. Folgende Primer wurden verwendet: (das Stop-Codon ist unterstrichen, das erste Phenylalanin-Codon des gereiften Gens ist fett gedruckt)
forward 5'-**TTT**GTCTTGGCTCCCAAAAAGCCA
reverse 5'-TTAAAAAGCAAAACGTTCCAACTTGAGCAATCC

Für die PCR Amplifikation wurde folgendes Zeit/Temperatur-Programm durchgeführt: 2 min bei 94 °C denaturiert und danach 15 Zyklen von 15 sec bei 94 °C, 30 sec bei 50 °C, 90 sec bei 72 °C plus 5 sec pro Zyklus für die Extensionsperiode von Zyclus 11 und als letzten Schritt 7 min bei 72 °C.

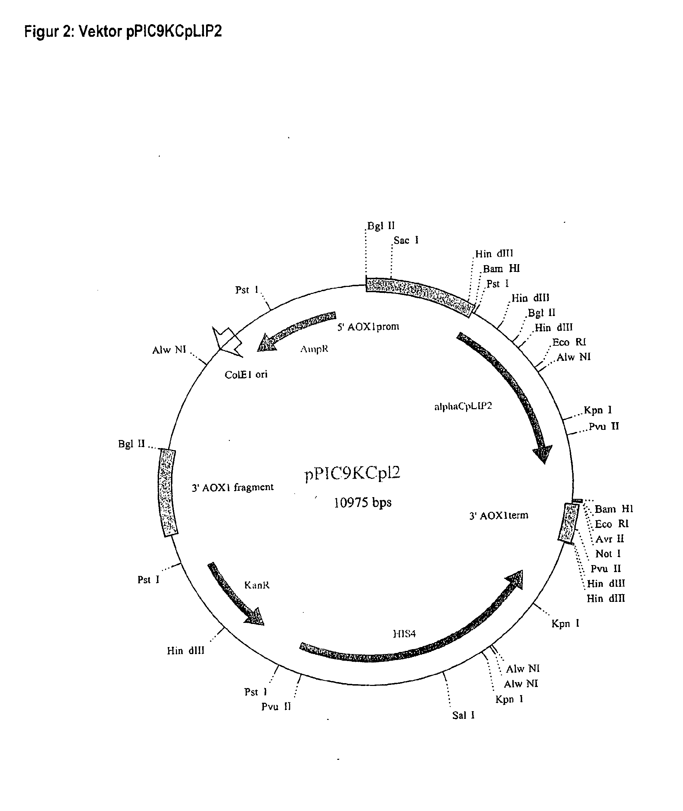

Nach der Amplifizierung wurde das erhaltene Fragment mit T4-Polynucleotid-Lipase phosphoryliert und mit T4-DNA-Polymerase abgestumpft. Das Fragment wurde anschliessend mit einem mit SnaBI digestierten pPIC9K Plasmid ligiert und man erhielt den Vektor pPIC9KCpLIP2 (replikatives Plasmid) **(****Figure 2****).** Die Abwesenheit von Mutationen wurde durch Sequenzierung der Inserts geprüft.

Die Transformation der Hefe-Spheroplasten wurde mit dem *Pichia*-Expressions kit der Firma Invitrogen (Groningen Niederlande) durchgeführt. Die Transformationsfrequenz betrug 10³ Transformationen pro µg DNA.

Ein selektierter Transformant wurde mit einem synthetisches Medium beschrieben von Boze et al. in: (Boze H. et al.; Process Biochem, 2001, 36, 907-913) welchem 40 g/l Glycerol zugesetzt wurden, in einem Fermenter kultiviert. Nach der Wachstumsphase (nach 2500 min Fermentation) im batch Verfahren wurde im fed-batch-Verfahren reines Methanol (5 g/l) zugegeben um die Expression des Gens zu induzieren. Nach 4-tägiger Kultivierung mit einer hohen Zelldichte wurde der Überstand der Kulturbrühe durch Zentrifugation vom Rückstand getrennt. Der erhaltene Überstand enthielt 102000 U/l der rekombinanten Lipase/Acyltransferase mit einer spezifischen Aktivität von 80 U/mg Protein, Eine Aufkonzentration über Ultrafiltration mit 10000 kD cut-off Membranen ergaben eine Enzymkonzentration von 830000 U/l mit einer spezifischen Aktivität von 150 U/mg.

### 5. Beispiel: Expression einer modifizierten (His-tagged) LipaselAcyltransferase in Saccharomyces cerevisiae

Zur Expression der modifizierten gewünschten Nucleinsäuresequenz nach SEQ ID NO 3, welche die Fusion von 6-His-Peptid zum C-terminalen Ende der Sequenz des Polypeptides nach SEQ ID NO 2 ermöglicht, wurde zunächst mit der Restriktionsendonuciease *Bam*HI DNA partiell hydrolysiert. Mittels PCR wurden Primer konstruiert, welche die Nucleinsäure nach SEQ ID NO. 1 enthalten. Folgende Primer-Paare, die eine Extension der Nucleinsäure-Sequenz mit 6 Histidin-Codons ermöglicht, wurden verwendet (Start und Stop-Codon sind unterstrichen; *Bam*HI-Restriktionsseite ist fett gedruckt, His-Codons sind kursiv gedruckt):
forward 5'-CTC**GGATCC**ATGCGTTACTTTGCTATTGC
reverse 5'-CAC**GGATCC**TTA*ATGATGATGATGATGATG*AAAAGCAAAACGTTCCAACTTGAGCAATCC

Für die PCR Amplifikation wurde folgendes Zeit/Temperatur-Programm durchgeführt: 5 min bei 95 °C denaturiert und danach 30 Zyklen von 1 min bei 95 °C, 1 min bei 50 °C, 1 min bei 72 °C und als letzten Schritt 10 min bei 72 °C.

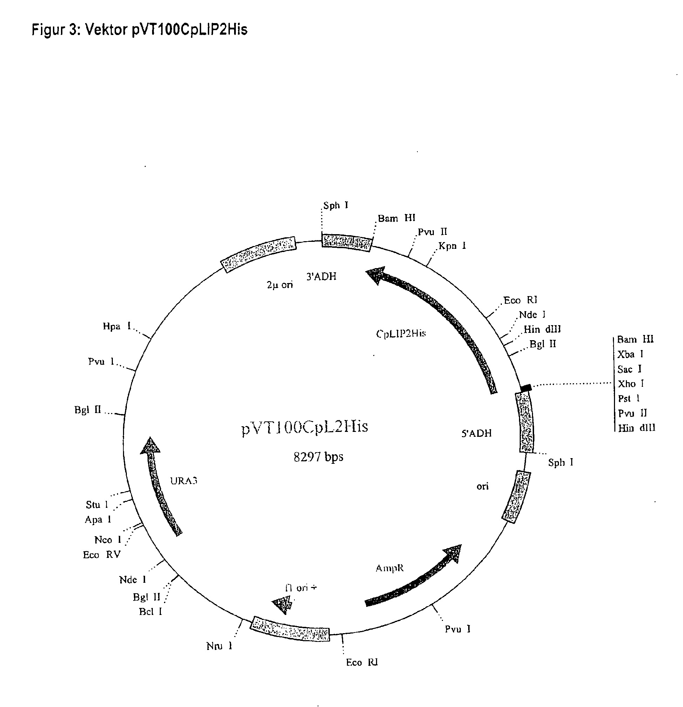

Die Fragmente aus der PCR wurden mit der Restriktionsendonuclease *Bam*HI digestiert und anschliessend in den mit der Restriktionsendonuclease *Bam*HI geschnittenen Vektor pVT100-U zum Plasmid ligiert. Erhalten wurde der Vektor pVT100CpLIP2His nach **Figur 3** (integratives Plasmid). Die Transformation von *Saccharomyces cerevisiae* W303-1a und die Expression des Gens wurde entsprechend Beispiel 3 durchgeführt.

Der selektierte Transfomant wurde in Schüttelkolben bei 28 °C in YPD Medium ((YPD = 10 g/l Yeast extract [Difco], 20 g/l Bacto peptone [Difco], 60 g/l Glukose, 150 mg/l Leucin, 100 mg/l Adenine, 100 mg/l Histidin und 100 mg/l Tryptophan) kultiviert. Die Kulturbrühe wurde nach 36 Stunden Fermentation geerntet und der Überstand der Kulturlösung durch Zentrifugieren vom Rückstand getrennt. Der Überstand enthielt 3100 U der rekombinanten his-tagged Lipase/Acyltransferase pro Liter und eine spezifische Aktivität von 0,25 U/mg Protein. Ionen-chelatierende Eigenschaften wurden ausgenutzt für eine Aufreinigung in einem Schritt. Dazu wurde Ni-NitriloTriacetic-Acid Agarose Affinitätsgel der Firma Qiagen wie vom Hersteller beschrieben verwendet. Man erhielt 26 % des Enzyms mit einer spezifischen Aktivität von 150 U/mg Protein.

## Patentansprüche

1. Polypeptide mit Lipase/Acyltransferase Aktivität mit einer Aminosäuresequenz, welche zu der in SEQ ID No. 2 angegebenen Aminosäuresequenz wenigstens 80 % Identität besitzt.

2. Polypeptide mit Lipase/Acyltransferase Aktivität mit einer Aminosäuresequenz, welche zu der in SEQ ID No. 2 angegebenen Aminosäuresequenz in den Positionen 190 bis 390 mindestens zu 80 % identisch sind.

3. Polypeptide mit Lipase/Acyltransferase Aktivität mit einer Aminosäuresequenz, welche zu der in SEQ ID No. 2 angegebenen Aminosäuresequenz in den Positionen 190 bis 390 mindestens zu 96 % identisch sind.

4. Polypeptide mit Lipase/Acyltransferase Aktivität mit einer Aminosäuresequenz, welche zu der in SEQ ID No. 2 angegebenen Aminosäuresequenz identisch sind.

5. Polypeptide nach einem der Ansprüche 1 bis 4 **dadurch gekennzeichnet, dass** sie glycosyliert vorliegen.

6. Polypeptide nach einem der Ansprüche 1 bis 4 **dadurch gekennzeichnet, dass** sie mit einem weiteren Peptid verknüpft sind.

7. Polypeptide nach Anspruch 6, **dadurch gekennzeichnet, dass** es sich bei dem weiteren Peptid um einen Marker, vorzugsweise um his-tag handelt.

8. Polypeptide mit einer Aminosäuresequenz, welche zu der in SEQ ID No. 4 angegebenen Aminosäuresequenz wenigstens 80 % Identität besitzt.

9. Polypeptide mit einer Aminosäuresequenz, welche zu der in SEQ ID No. 4 angegebenen Aminosäuresequenz zu 100 % identisch ist.

10. Polypeptidfragmente oder durch Deletionsmutation erhältliche Polypeptide mit Lipase/Acyltransferase Aktivität gemäß einem der Ansprüche 1 bis 9.

11. Polypeptide mit Lipase/Acyltransferase Aktivität gemäß der Ansprüche 1 bis 10 deren reine Aminosäurekette chemisch modifiziert worden ist.

12. Polypeptide nach mindestens einem der Ansprüche 1 bis 11 **dadurch gekennzeichnet, dass** sie natürlicherweise aus einem Mikroorganismus erhältlich sind.

13. Polypeptide nach Anspruch 12 **dadurch gekennzeichnet, dass** es sich bei dem Mikroorganismus um eukaryontische Pilze, vorzugsweise Hefepilze, handelt.

14. Polypeptide nach Anspruch 12 **dadurch gekennzeichnet**, das sie erhältlich sind aus Mikroorganismen, ausgewählt aus der Gruppe, die gebildet wird von *Candida parapsilosis,* Candida antarctica (Trychosporon oryzae, Pseudozyma antarctica), Candida glabrata, *Candida albicans, Candida maltosa, Candida tropicalis, Candida viswanathii, Issatchenkia* orientalis (Candida krusei), Kluyveromyces marxianus (C. kefyr, C. pseudotropicalis), Pichia guilliemondii (Candida guilliermondii), Geotrichum candidum, Fusarium solani und Aeromonas aerophila.

15. Für ein Polypeptid mit Lipase/Acyltransferase Aktivität kodierende Nucleinsäuren, deren Nucleotidsequenzen zu der in SEQ ID No. 1 angegebenen Nucleotidsequenz zu 100 % identisch sind, insbesondere über den Teilbereich, der den Aminosäuren 190 bis 390 gemäß der SEQ ID No. 2 entspricht.

16. Nucleinsäuren kodierend für eine Aminosäuresequenz, welche zu der in SEQ ID No. 2 angegebenen Aminosäuresequenz wenigstens 80 % Identität besitzt, wobei die Aminosäuresequenz ein Polypeptid mit Lipase/Acyltransferase Aktivität darstellt.

17. Nucleinsäuren kodierend für eine Aminosäuresequenz, welche zu der in SEQ ID No. 2 angegebenen Aminosäuresequenz in den Positionen 190 bis 390 mindestens zu 96 % identisch ist, wobei die Aminosäuresequenz ein Polypeptid mit Lipase/Acyltransferase Aktivität darstellt.

18. Nucleinsäuren, die für eines der in den Ansprüchen 1 bis 14 bezeichneten Polypeptide oder Derivate kodiert.

19. Für ein Polypeptid mit Lipase/Acyltransferase Aktivität kodierende Nucleinsäuren, deren Nucleotidsequenz zu der in SEQ ID No. 3 angegebenen Nucleotidsequenz identisch ist,

20. Nucleinsäuren kodierend für eine Aminosäuresequenz, welche zu der in SEQ ID No. 4 angegebenen Aminosäuresequenz wenigstens 80 % Identität besitzt, wobei die Aminosäuresequenz ein Polypeptid mit Lipase/Acyltransferase Aktivität darstellt.

21. Vektor, der eine in den Ansprüchen 15 bis 20 bezeichnete Nucleinsäure enthält, die für eines der in den Ansprüchen 1 bis 14 bezeichneten Polypeptide oder Derivate kodiert.

22. Klonierungsvektor gemäß Anspruch 21.

23. Expressionsvektor gemäß Anspruch 21.

24. Zelle, die einen Vektor nach einem der Ansprüche 21 bis 23 enthält.

25. Transformierte Wirtszelle, die eines der in den Ansprüchen 1 bis 14 bezeichneten Polypeptide oder Derivate exprimiert oder zu dessen Expression angeregt werden kann, unter Einsatz eines Expressionsvektors gemäß Anspruch 23.

26. Transformierte Wirtszelle nach Anspruch 25, enthaltend eine Nucleinsäure welche für eine Aminosäuresequenz kodiert, welche zu der in SEQ ID No. 2 angegebenen Aminosäuresequenz 100 % Identität besitzt.

27. Transformierte Wirtszelle nach Anspruch 25 und/oder 26, enthaltend eine Nucleinsäure, welche für eine Aminosäuresequenz kodiert, zu der in SEQ ID No. 2 angegebenen Aminosäuresequenz wenigstens 80 % Identität besitzt.

28. Transformierte Wirtszelle nach Anspruch 25, enthaltend eine Nucleinsäure, welche für eine Aminosäuresequenz kodiert, zu der in SEQ ID No. 4 angegebenen Aminosäuresequenz 100 % Identität besitzt.

29. Transformierte Wirtszelle nach Anspruch 25 und/oder 26, enthaltend eine Nucleinsäure, welche für eine Aminosäuresequenz kodiert, die zu der in SEQ ID No. 4 angegebenen Aminosäuresequenz wenigstens 80 % Identität besitzt.

30. Transformierte Wirtszellen nach einem der Ansprüche 25 bis 29, **dadurch gekennzeichnet, dass** es sich bei den zu transformierenden Wirtszellen um Wirtszellen aus Mikroorganismen handelt.

31. Transformierte Wirtszellen nach einem der Ansprüche 25 bis 30, **dadurch gekennzeichnet, dass** es sich bei den zu transformierenden Wirtszellen um Wirtszellen auf Mikroorganismen handelt, die ausgewählt sind aus der Gruppe, die gebildet wird von *Candida parapsilosis,* Saccharamyces cerevisiae, Schizosaccharomyces pombe, Pichia pastoris, Pichia boidinii, Pichia stipitis, Hansenula polymorpha, Kluyveromyces lactis, Schwanniomyces *castellii,* Yarrowia lipolytica, Escherishia coli, Bacilius subtilis, Bacillus amylolichefaciens, Bacillus stearothermophilus, Bacillus licheniformis, Lactococcus lactis, Streptococcus lactis, Lactobacillus bulgaricus, Aspergillus orizae, Aspergillus niger, Trichoderma reesei, Mucor sp. und Rhizopus sp.

32. Verfahren zur Herstellung eines Polypeptids nach mindestens einem der Ansprüche 1 bis 14, unter Verwendung einer Nucleinsäure, die für eine Aminosäuresequenz kodiert, welche zu der in SEQ ID No. 2 angegebenen Aminosäuresequenz wenigstens 80 % Identität besitzt und/oder unter Verwendung eines Vektors gemäß einem der Ansprüche 21 bis 23 und/oder unter Verwendung einer transformierten Wirtszelle gemäß einem der Ansprüche 25 bis 31 oder unter Verwendung einer Zelle, die dieses natürlicherweise bildet.

33. Verwendung von natürlichen und/oder rekombinanten Mikroorganismen enthaltend eine Nucleinsäure zur Herstellung eines Polypeptides nach mindestens einem der Ansprüche 1 bis 14.

34. Verwendung einer Nucleinsäure gemäß den Ansprüchen 15 bis 20 und/oder Verwendung von Aminosäuresequenzen welche zu der in SEQ ID No. 2 angegebenen Aminosäuresequenz wenigstens 80 % Identität besitzt zum Auffinden neuer Acyltransferasen

35. Verwendung einer Nucleinsäure gemäß den Ansprüchen 15 bis 20 und/oder Verwendung von Aminosäuresequenzen, welche zu der in SEQ ID No. 4 angegebenen Aminosäuresequenz wenigstens 80 % Identität besitzt zum Auffinden neuer Acyltransferasen.

36. Verwendung von Polypeptiden nach Anspruch 1 bis 14 als Katalysatoren bei Acyltransferreaktionen, insbesondere bei Reaktionen die ausgewählt sind aus der Gruppe die gebildet wird von Alkoholyse von Esther, Alkoholyse von Thioestern, Thiolyse von Estern, Aminolyse eines Esters mit Hydroxylaminen oder Hydrazinen; Reaktion eines Esthers mit Hydrogen-Peroxiden und enantioselektive Synthese von Estern, Thioestern, Lactonen durch Alkoholyse.

## Claims

1. Polypeptides with lipase/acyltransferase activity with an amino acid sequence which has at least 80% identity to the amino acid sequence reported in SEQ ID No. 2.

2. Polypeptides with lipase/acyltransferase activity with an amino acid sequence which has at least 80% identity to the amino acid sequence reported in SEQ ID No. 2 at positions 190 to 390.

3. Polypeptides with lipase/acyltransferase activity with an amino acid sequence which has at least 96% identity to the amino acid sequence reported in SEQ ID No. 2 at positions 190 to 390.

4. Polypeptides with lipase/acyltransferase activity with an amino acid sequence which is identical to the amino acid sequence reported in SEQ ID No. 2.

5. Polypeptides according to any of Claims 1 to 4, **characterized in that** they are present in glycosylated form.

6. Polypeptides according to any of Claims 1 to 4, **characterized in that** they are linked to another peptide.

7. Polypeptides according to Claim 6, **characterized in that** the further peptide is a marker, preferably a his-tag.

8. Polypeptides with an amino acid sequence which has at least 80% identity to the amino acid sequence reported in SEQ ID No. 4.

9. Polypeptides with an amino acid sequence which has at least 100% identity to the amino acid sequence reported in SEQ ID No. 4.

10. Polypeptide fragments, or polypeptides obtainable by deletion mutation, with lipase/acyltransferase activity according to one of Claims 1 to 9.

11. Polypeptides with lipase/acyltransferase activity according to Claims 1 to 10, whose pure amino acid chain has been chemically modified.

12. Polypeptides according to at least one of Claims 1 to 11, **characterized in that** they are obtainable naturally from a microorganism.

13. Polypeptides according to Claim 12, **characterized in that** the microorganism takes the form of eukaryotic fungi, preferably yeast fungi.

14. Polypeptides according to Claim 12, **characterized in that** they are obtainable from microorganisms selected from the group formed by Candida parapsilosis, Candida antarctica (Trychosporon oryzae, Pseudozyma antarctica), Candida glabrata, Candida albicans, Candida maltosa, Candida tropicalis, Candida viswanathii, Issatchenkia orientalis (Candida krusei), Kluyveromyces marxianus (C. kefyr, C. pseudotropicalis), Pichia guilliemondii (Candida guilliermondii), Geotrichum candidum, Fusarium solani and Aeromonas aerophila.

15. Nucleic acids coding for a polypeptide with lipase/acyltransferase activity, whose nucleotide sequences are 100% identical to the nucleotide sequence reported in SEQ ID No. 1, especially over the partial region which corresponds to amino acids 190 to 390 according to SEQ ID No. 2.

16. Nucleic acids coding for an amino acid sequence which has at least 80% identity to the amino acid sequence reported in SEQ ID No. 2, the amino acid sequence standing for a polypeptide with lipase/acyltransferase activity.

17. Nucleic acids coding for an amino acid sequence which has at least 96% identity to the amino acid sequence in positions 190 to 390, the amino acid sequence standing for a polypeptide with lipase/acyltransferase activity.

18. Nucleic acids which code for one of the polypeptides or derivatives designated in Claims 1 to 14.

19. Nucleic acids coding for a polypeptide with lipase/acyltransferase activity, whose nucleotide sequence is identical to the nucleotide sequence reported in SEQ ID No. 3.

20. Nucleic acids coding for an amino acid sequence which has at least 80% identity to the amino acid sequence reported in SEQ ID No. 4, the amino acid sequence standing for a polypeptide with lipase/acyltransferase activity.

21. Vector which contains one of the nucleic acids designated in Claims 15 to 20, which codes for one of the polypeptides or derivatives designated in Claims 1 to 14.

22. Cloning vector according to Claim 21.

23. Expression vector according to Claim 21.

24. Cell which contains a vector according to any of Claims 21 to 23.

25. Transformed host cell which expresses, or which can be stimulated to express, one of the polypeptides or derivatives designated in Claims 1 to 14, using an expression vector according to Claim 23.

26. Transformed host cell according to Claim 25, containing a nucleic acid which codes for an amino acid sequence with 100% identity to the amino acid sequence reported in SEQ ID No. 2.

27. Transformed host cell according to Claim 25 and/or 26, containing a nucleic acid which codes for an amino acid sequence with at least 80% identity to the amino acid sequence reported in SEQ ID No. 2.

28. Transformed host cell according to Claim 25, containing a nucleic acid which codes for an amino acid sequence with 100% identity to the amino acid sequence reported in SEQ ID No. 4.

29. Transformed host cell according to Claim 25 and/or 26, containing a nucleic acid which codes for an amino acid sequence with at least 80% identity to the amino acid sequence reported in SEQ ID No. 4.

30. Transformed host cells according to any of Claims 25 to 29, **characterized in that** the host cells to be transformed take the form of host cells from microorganisms.

31. Transformed host cells according to one of Claims 25 to 30, **characterized in that** the host cells to be transformed take the form of host cells of microorganisms which are selected from the group formed by Candida parapsilosis, Saccharamyces cerevisiae, Schizosaccharomyces pombe, Pichia pastoris, Pichia boidinii, Pichia stipitis, Hansenula polymorpha, Kluyveromyces lactis, Schwanniomyces castellii, Yarrowia lipolytica, Escherishia coli, Bacillus subtilis, Bacillus amylolichefaciens, Bacillus stearothermophilus, Bacillus licheniformis, Lactococcus lactis, Streptococcus lactis, Lactobacillus bulgaricus, Aspergillus orizae, Aspergillus niger, Trichoderma reesei, Mucor sp. and Rhizopus sp.

32. Process for the preparation of a polypeptide according to at least one of Claims 1 to 14 using a nucleic acid which codes for an amino acid sequence which has at least 80% identity to the amino acid sequence reported in SEQ ID No. 2 and/or using a vector according to any of Claims 21 to 23 and/or using a transformed host cell according to any of Claims 25 to 31 or using a cell which forms this naturally.

33. Use of natural and/or recombinant microorganisms containing a nucleic acid for the preparation of a polypeptide according to at least one of Claims 1 to 14.

34. Use of a nucleic acid according to Claims 15 to 20 and/or use of amino acid sequences with at least 80% identity to the amino acid sequence reported in SEQ ID No. 2 for finding novel acyltransferases.

35. Use of a nucleic acid according to Claims 15 to 20 and/or use of amino acid sequences with at least 80% identity to the amino acid sequence reported in SEQ ID No. 4 for finding novel acyltransferases.

36. Use of polypeptides according to Claims 1 to 14 as catalysts in acyltransferase reactions, in particular in reactions which are selected from the group formed by the alcoholysis of esters, the alcoholysis of thioesters, the thiolysis of esters, the aminolysis of an ester with hydroxylamines or hydrazines; the reaction of an ester with hydrogen peroxides and the enantioselective synthesis of esters, thioesters, lactones by alcoholysis.

## Revendications

1. Polypeptides à activité de lipase/acyltransférase, avec une séquence d'acides aminés qui présente, par rapport à la séquence d'acides aminés indiquée dans SEQ ID N°2, une identité d'au moins 80 %.

2. Polypeptides à activité de lipase/acyltransférase, avec une séquence d'acides aminés qui est identique à au moins 80 % à la séquence d'acides aminés indiquée dans SEQ ID N°2, sur les positions 190 à 390.

3. Polypeptides à activité de lipase/acyltransférase, avec une séquence d'acides aminés qui est identique à au moins 96 % à la séquence d'acides aminés indiquée dans SEQ ID N°2, sur les positions 190 à 390.

4. Polypeptides à activité de lipase/acyltransférase, avec une séquence d'acides aminés qui est identique à la séquence d'acides aminés indiquée dans SEQ ID N°2.

5. Polypeptides selon l'une des revendications 1 à 4, **caractérisés en ce qu'**ils se présentent sous forme glycosylée.

6. Polypeptides selon l'une des revendications 1 à 4, **caractérisés en ce qu'**ils sont reliés à un autre polypeptide.

7. Polypeptides selon la revendication 6, **caractérisés en ce que**, pour ce qui concerne l'autre peptide, il s'agit d'un marqueur, de préférence de his-tag.

8. Polypeptides avec une séquence d'acides aminés qui présente, par rapport à la séquence d'acides aminés indiquée dans SEQ ID N°4, une identité d'au moins 80 %.

9. Polypeptides avec une séquence d'acides aminés qui est identique à 100 % à la séquence d'acides aminés indiquée dans SEQ ID N°4.

10. Fragments polypeptidiques ou polypeptides que l'on peut obtenir par une mutation par délétion, à activité de lipase/acyltransférase selon l'une des revendications 1 à 9.

11. Polypeptides à activité de lipase/acyltransférase selon les revendications 1 à 10, dont la chaîne d'acides aminés pure a été chimiquement modifiée.

12. Polypeptides selon au moins l'une des revendications 1 à 11, **caractérisés en ce qu'**on peut les obtenir de façon naturelle à partir d'un microorganisme.

13. Polypeptides selon la revendication 12, **caractérisés en ce que**, pour ce qui concerne le microorganisme, il s'agit de champignons eucaryotes, de préférence de champignons de type levure.

14. Polypeptides selon la revendication 12, **caractérisés en ce qu'**on peut les obtenir à partir de microorganismes choisis dans le groupe formé par Candida parapsilosis, Candida antarctica (Trychosporon oryzae, Pseudozyma antartica), Candida glabrata, Candida albicans, Candida maltosa, Candida tropicalis, Candida viswanathii, Issatchenkia orientalis (Candida krusei), Kluyveromyces marxianus (C. kefyr, C. pseudotropicalis), Pichia guilliemondii (Candida guillermondii), Geotrichum candidum, Fusarium solani et Aeromonas aerophila.

15. Acides nucléiques codant pour un polypeptide à activité de lipase/transférase, dont les séquences d'acides nucléiques sont identiques à 100 % à la séquence nucléotidique indiquée dans SEQ ID N°1, en particulier dans la région partielle qui correspond aux acides aminés 190 à 390 selon SEQ ID N°2.

16. Acides nucléiques codant pour une séquence d'acides aminés qui présente une identité d'au moins 80 % avec la séquence d'acides aminés indiquée dans SEQ ID N°2, la séquence d'acides aminés représentant un polypeptide à activité de lipase/acyltransférase.

17. Acides nucléiques codant pour une séquence d'acides aminés qui est identique à au moins 96 % avec la séquence d'acides aminés indiquée dans SEQ ID N°2 dans les positions 190 à 390, la séquence d'acides aminés représentant un polypeptide à activité de lipase/acyltransférase.

18. Acides nucléiques qui codent pour l'un des polypeptides ou dérivés désignés dans les revendications 1 à 14.

19. Acides nucléiques codant pour un polypeptide à activité de lipase/acyltransférase, dont la séquence nucléotidique est identique à la séquence nucléotidique indiquée dans SEQ ID N°3.

20. Acides nucléiques codant pour une séquence d'acides aminés qui présente une identité d'au moins 80 % avec la séquence d'acides aminés indiquée dans SEQ ID N°4, la séquence d'acides aminés représentant un polypeptide à activité de lipase/acyltransférase.

21. Vecteur qui contient un acide nucléique désigné dans les revendications 15 à 20, qui code pour l'un des polypeptides ou dérivés désignés dans les revendications 1 à 14.

22. Vecteur de clonage selon la revendication 21.

23. Vecteur d'expression selon la revendication 21.

24. Cellule qui contient un vecteur selon l'une des revendications 21 à 23.

25. Cellule hôte transformée qui exprime l'un des polypeptides ou dérivés désignés dans les revendications 1 à 14, ou qui peut être excitée pour son expression, par utilisation d'un vecteur d'expression selon la revendication 23.

26. Cellule hôte transformée selon la revendication 25, contenant un acide nucléique qui code pour une séquence d'acides aminés qui présente une identité de 100 % avec la séquence d'acides aminés indiquée dans SEQ ID N°2.

27. Cellule hôte transformée selon la revendication 25 et/ou 26, contenant un acide nucléique qui code pour une séquence d'acides aminés qui présente une identité d'au moins 80 % avec la séquence d'acides aminés indiquée dans SEQ ID N°2.

28. Cellule hôte transformée selon la revendication 25, contenant un acide nucléique qui code pour une séquence d'acides aminés qui présente une identité de 100 % avec la séquence d'acides aminés indiquée dans SEQ ID N°4.

29. Cellule hôte transformée selon la revendication 25 et/ou 26, contenant un acide nucléique qui code pour une séquence d'acides aminés qui présente une identité d'au moins 80 % avec la séquence d'acides aminés indiquée dans SEQ ID N°4.

30. Cellules hôtes transformées selon l'une des revendications 25 à 29, **caractérisées en ce que**, pour ce qui concerne les cellules hôtes à transformer, il s'agit de cellules hôtes provenant de microorganismes.

31. Cellules hôtes transformées selon l'une des revendications 25 à 30, **caractérisées en ce que**, pour ce qui concerne les cellules hôtes à transformer, il s'agit de cellules hôtes provenant de microorganismes qui sont choisies dans le groupe formé par Candida parapsilosis, Saccharomyces cerevisiae, Schizosaccharomyces pombe, Pichia pastoris, Pichia boidinii, Pichia stipitis, Hanseluna polymorpha, Kluyveromyces lactis, Schwanniomyces castellii, Yarrowia lipolytica, Escherichia coli, Bacillus subtilis, Bacillus amylolichefaciens, Bacillus stearothermophilus, Bacillus licheniformis, Lactococcus lactis, Streptococcus lactis, Lactobacillus bulgaricus, Aspergillus orizae, Aspergillus niger, Trichoderma reesii, Mucor sp. et Rhizopus sp.

32. Procédé de préparation d'un polypeptide selon au moins l'une des revendications 1 à 14, par utilisation d'un acide nucléique qui code pour une séquence d'acides aminés qui présente une identité d'au moins 80 % avec la séquence d'acides aminés indiquée dans SEQ ID N°2, et/ou par utilisation d'un vecteur selon l'une des revendications 21 à 23 et/ou par utilisation d'une cellule hôte transformée selon l'une des revendications 25 à 31, ou par utilisation d'une cellule qui la forme naturellement.

33. Utilisation de microorganismes naturels et/ou recombinants contenant un acide nucléique pour la préparation d'un polypeptide selon au moins l'une des revendications 1 à 14.

34. Utilisation d'un acide nucléique selon les revendications 15 à 20 et/ou utilisation de séquences d'acides aminés qui présentent une identité d'au moins 80 % avec la séquence d'acides aminés indiquée dans SEQ ID N°2, pour la découverte de nouvelles acyltransférases.

35. Utilisation d'un acide nucléique selon les revendications 15 à 20 et/ou utilisation de séquences d'acides aminés qui présentent une identité d'au moins 80 % avec la séquence d'acides aminés indiquée dans SEQ ID N°4, pour la découverte de nouvelle acyltransférases.

36. Utilisation de polypeptides selon les revendications 1 à 14, en tant que catalyseurs dans des réactions de transfert de groupes acyle, en particulier lors de réactions qui sont choisies dans le groupe formé par l'alcoolyse des esters, l'alcoolyse des thioesters, la thiolyse des esters, l'aminolyse d'un ester avec des hydroxylamines ou des hydrazines ; la réaction d'un ester avec des peroxydes d'hydrogène et la synthèse énantio-sélective d'esters, de thioesters, de lactones par alcoolyse.


### SEQUENZPROTOKOLL

<110> Cognis Deutschland GmbH
<120> Lipase/Acyltransferase
<130> C2425
<140>
   <141>
<160> 4
<170> PatentIn Ver. 2.1
<210> 1
   <211> 1398
   <212> DNA
   <213> Candida parapsilosis
<220>
   <221> CDS
   <222> (1)..(1398)
<400> 1
   atg cgt tac ttt gct att gct ttc ttg ctc atc aat acc att tca gct      48
   Met Arg Tyr Phe Ala Ile Ala Phe Leu Leu Ile Asn Thr Ile Ser Ala
    1               5                  10                  15
   ttt gtc ttg gct ccc aaa aag cca tct caa gac gat ttc tac act cca      96
   Phe Val Leu Ala Pro Lys Lys Pro Ser Gln Asp Asp Phe Tyr Thr Pro
               20                  25                  30
   cca caa ggt tat gaa gct caa cct ctt ggt tct att ttg aaa aca aga      144
   Pro Gln Gly Tyr Glu Ala Gln Pro Leu Gly Ser Ile Leu Lys Thr Arg
           35                  40                  45
   aac gtc ccc aat cca ttg act aat gtt ttc act cca gtt aaa gtt caa      192
   Asn Val Pro Asn Pro Leu Thr Asn Val Phe Thr Pro Val Lys Val Gln
       50                  55                  60
   aat gca tgg caa tta ttg gtt aga tct gaa gat aca ttt ggt aac cca      240
   Asn Ala Trp Gln Leu Leu Val Arg Ser Glu Asp Thr Phe Gly Asn Pro
   65                  70                  75                  80
   aac gcc ata gtc act acc att att caa cct ttc aat gct aaa aag gat      288
   Asn Ala Ile Val Thr Thr Ile Ile Gln Pro Phe Asn Ala Lys Lys Asp
                   85                  90                  95
   aag ctt gtt tct tat caa aca ttt gaa gat tct ggt aaa ttg gat tgt      336
   Lys Leu Val Ser Tyr Gln Thr Phe Glu Asp Ser Gly Lys Leu Asp Cys
               100                 105                 110
   gct cca tca tat gct att caa tat gga tcg gac att tcg act ttg acc      384
   Ala Pro Ser Tyr Ala Ile Gln Tyr Gly Ser Asp Ile Ser Thr Leu Thr
           115                 120                 125
   act caa ggt gaa atg tac tac atc tct gct tta tta gat caa ggt tac      432
   Thr Gln Gly Glu Met Tyr Tyr Ile Ser Ala Leu Leu Asp Gln Gly Tyr
       130                 135                 140
   tat gtt gtc act cct gat tac gag ggt cca aag agt aca ttc act gta      480
   Tyr Val Val Thr Pro Asp Tyr Glu Gly Pro Lys Ser Thr Phe Thr Val
   145                 150                 155                 160
   ggg ttg caa tca gga aga gct act ttg aat tcg ctt aga gct act ttg    528
   Gly Leu Gln Ser Gly Arg Ala Thr Leu Asn Ser Leu Arg Ala Thr Leu
                   165                 170                 175
   aaa tca gga aac ttg act ggt gtt tca tca gac gct gag aca tta ttg    576
   Lys Ser Gly Asn Leu Thr Gly Val Ser Ser Asp Ala Glu Thr Leu Leu
               180                 185                 190
   tgg ggt tat tca gga gga agt ctt gct tca gga tgg gct gct gct ata    624
   Trp Gly Tyr Ser Gly Gly Ser Leu Ala Ser Gly Trp Ala Ala Ala Ile
           195                 200                 205
   caa aaa gaa tat gct cca gag ttg agt aaa aac ttg ctt ggt gct gca    672
   Gln Lys Glu Tyr Ala Pro Glu Leu Ser Lys Asn Leu Leu Gly Ala Ala
       210                 215                 220
   ctt ggt gga ttc gtt aca aac att act gcc act gct gaa gct gtt gat    720
   Leu Gly Gly Phe Val Thr Asn Ile Thr Ala Thr Ala Glu Ala Val Asp
   225                 230                 235                 240
   agt ggt cca ttt gca gga atc atc tcc aat gca ttg gct ggt att gga    768
   Ser Gly Pro Phe Ala Gly Ile Ile Ser Asn Ala Leu Ala Gly Ile Gly
                   245                 250                 255
   aat gaa tac cct gat ttc aaa aac tat ctt ttg aaa aaa gtg tca cca    816
   Asn Glu Tyr Pro Asp Phe Lys Asn Tyr Leu Leu Lys Lys Val Ser Pro
               260                 265                 270
   ttg ctt tca atc act tat cgt ttg gga aac act cac tgt ttg ctt gat    864
   Leu Leu Ser Ile Thr Tyr Arg Leu Gly Asn Thr His Cys Leu Leu Asp
           275                 280                 285
   ggt ggt att gct tat ttc ggt aaa tca ttc ttt tcc aga att att aga    912
   Gly Gly Ile Ala Tyr Phe Gly Lys Ser Phe Phe Ser Arg Ile Ile Arg
       290                 295                 300
   tat ttc cct gat gga tgg gat ctt gtc aac caa gaa cct atc aaa acc    960
   Tyr Phe Pro Asp Gly Trp Asp Leu Val Asn Gln Glu Pro Ile Lys Thr
   305                 310                 315                 320
   atc ttg caa gat aat gga ttg gtt tac caa cca aag gac ttg acc cca    1008
   Ile Leu Gln Asp Asn Gly Leu Val Tyr Gln Pro Lys Asp Leu Thr Pro
                   325                 330                 335
   caa att cca tta ttc atc tac cac ggt acc ttg gat gca att gtc ccc    1056
   Gln Ile Pro Leu Phe Ile Tyr His Gly Thr Leu Asp Ala Ile Val Pro
               340                 345                 350
   att gtc aac tca aga aag aca ttc caa caa tgg tgt gat tgg gga ctc    1104
   Ile Val Asn Ser Arg Lys Thr Phe Gln Gln Trp Cys Asp Trp Gly Leu
           355                 360                 365
   aaa tct ggt gaa tat aat gaa gat ttg acc aat gga cac att act gaa    1152
   Lys Ser Gly Glu Tyr Asn Glu Asp Leu Thr Asn Gly His Ile Thr Glu
       370                 375                 380
   tca att gtg ggt gca cca gct gct ttg act tgg att atc aat cgt ttc    1200
   Ser Ile Val Gly Ala Pro Ala Ala Leu Thr Trp Ile Ile Asn Arg Phe
   385                 390                 395                 400
   aat gga cag cct cca gtt gat gga tgt caa cat aat gtg aga gct tca    1248
   Asn Gly Gln Pro Pro Val Asp Gly Cys Gln His Asn Val Arg Ala Ser
                   405                 410                 415
   aac ttg gaa tat cca gga act cca caa tca atc aag aat tac ttt gaa    1296
   Asn Leu Glu Tyr Pro Gly Thr Pro Gln Ser Ile Lys Asn Tyr Phe Glu
               420                 425                 430
   gct gca ttg cac gca att ttg ggc ttt gat ttg ggt cca gat gtt aag    1344
   Ala Ala Leu His Ala Ile Leu Gly Phe Asp Leu Gly Pro Asp Val Lys
           435                 440                 445
   aga gat aag gtt act ttg ggc gga ttg ctc aag ttg gaa cgt ttt gct    1392
   Arg Asp Lys Val Thr Leu Gly Gly Leu Leu Lys Leu Glu Arg Phe Ala
       450                 455                 460
   ttt tag                                                            1398
   Phe
   465
<210> 2
   <211> 465
   <212> PRT
   <213> Candida parapsilosis
<400> 2
   Met Arg Tyr Phe Ala Ile Ala Phe Leu Leu Ile Asn Thr Ile Ser Ala
   1               5                   10                  15
   Phe Val Leu Ala Pro Lys Lys Pro Ser Gln Asp Asp Phe Tyr Thr Pro
               20                  25                  30
   Pro Gln Gly Tyr Glu Ala Gln Pro Leu Gly Ser Ile Leu Lys Thr Arg
           35                  40                  45
   Asn Val Pro Asn Pro Leu Thr Asn Val Phe Thr Pro Val Lys Val Gln
       50                  55                  60
   Asn Ala Trp Gln Leu Leu Val Arg Ser Glu Asp Thr Phe Gly Asn Pro
   65                  70                  75                  80
   Asn Ala Ile Val Thr Thr Ile Ile Gln Pro Phe Asn Ala Lys Lys Asp
                   85                  90                  95
   Lys Leu Val Ser Tyr Gln Thr Phe Glu Asp Ser Gly Lys Leu Asp Cys
               100                 105                 110
   Ala Pro Ser Tyr Ala Ile Gln Tyr Gly Ser Asp Ile Ser Thr Leu Thr
           115                 120                 125
   Thr Gln Gly Glu Met Tyr Tyr Ile Ser Ala Leu Leu Asp Gln Gly Tyr
       130                 135                 140
   Tyr Val Val Thr Pro Asp Tyr Glu Gly Pro Lys Ser Thr Phe Thr Val
   145                 150                 155                 160
   Gly Leu Gln Ser Gly Arg Ala Thr Leu Asn Ser Leu Arg Ala Thr Leu
                   165                 170                 175
   Lys Ser Gly Asn Leu Thr Gly Val Ser Ser Asp Ala Glu Thr Leu Leu
               180                 185                 190
   Trp Gly Tyr Ser Gly Gly Ser Leu Ala Ser Gly Trp Ala Ala Ala Ile
           195                 200                 205
   Gln Lys Glu Tyr Ala Pro Glu Leu Ser Lys Asn Leu Leu Gly Ala Ala
       210                 215                 220
   Leu Gly Gly Phe Val Thr Asn Ile Thr Ala Thr Ala Glu Ala Val Asp
   225                 230                 235                 240
   Ser Gly Pro Phe Ala Gly Ile Ile Ser Asn Ala Leu Ala Gly Ile Gly
                   245                 250                 255
   Asn Glu Tyr Pro Asp Phe Lys Asn Tyr Leu Leu Lys Lys Val Ser Pro
               260                 265                 270
   Leu Leu Ser Ile Thr Tyr Arg Leu Gly Asn Thr His Cys Leu Leu Asp
           275                 280                 285
   Gly Gly Ile Ala Tyr Phe Gly Lys Ser Phe Phe Ser Arg Ile Ile Arg
       290                 295                 300
   Tyr Phe Pro Asp Gly Trp Asp Leu Val Asn Gln Glu Pro Ile Lys Thr
   305                 310                 315                 320
   Ile Leu Gln Asp Asn Gly Leu Val Tyr Gln Pro Lys Asp Leu Thr Pro
                   325                 330                 335
   Gln Ile Pro Leu Phe Ile Tyr His Gly Thr Leu Asp Ala Ile Val Pro
               340                 345                 350
   Ile Val Asn Ser Arg Lys Thr Phe Gln Gln Trp Cys Asp Trp Gly Leu
           355                 360                 365
   Lys Ser Gly Glu Tyr Asn Glu Asp Leu Thr Asn Gly His Ile Thr Glu
       370                 375                 380
   Ser Ile Val Gly Ala Pro Ala Ala Leu Thr Trp Ile Ile Asn Arg Phe
   385                 390                 395                 400
   Asn Gly Gln Pro Pro Val Asp Gly Cys Gln His Asn Val Arg Ala Ser
                   405                 410                 415
   Asn Leu Glu Tyr Pro Gly Thr Pro Gln Ser Ile Lys Asn Tyr Phe Glu
               420                 425                 430
   Ala Ala Leu His Ala Ile Leu Gly Phe Asp Leu Gly Pro Asp Val Lys
           435                 440                 445
   Arg Asp Lys Val Thr Leu Gly Gly Leu Leu Lys Leu Glu Arg Phe Ala
       450                 455                 460
   Phe
   465
<210> 3
   <211> 1416
   <212> DNA
   <213> Candida parapsilosis
<220>
   <221> CDS
   <222> (1)..(1416)
<400> 3
   atg cgt tac ttt gct att gct ttc ttg ctc atc aat acc att tca gct    48
   Met Arg Tyr Phe Ala Ile Ala Phe Leu Leu Ile Asn Thr Ile Ser Ala
    1               5                  10                  15
   ttt gtc ttg gct ccc aaa aag cca tct caa gac gat ttc tac act cca    96
   Phe Val Leu Ala Pro Lys Lys Pro Ser Gln Asp Asp Phe Tyr Thr Pro
               20                  25                  30
   cca caa ggt tat gaa gct caa cct ctt ggt tct att ttg aaa aca aga    144
   Pro Gln Gly Tyr Glu Ala Gln Pro Leu Gly Ser Ile Leu Lys Thr Arg
           35                  40                  45
   aac gtc ccc aat cca ttg act aat gtt ttc act cca gtt aaa gtt caa    192
   Asn Val Pro Asn Pro Leu Thr Asn Val Phe Thr Pro Val Lys Val Gln
       50                  55                  60
   aat gca tgg caa tta ttg gtt aga tct gaa gat aca ttt ggt aac cca    240
   Asn Ala Trp Gln Leu Leu Val Arg Ser Glu Asp Thr Phe Gly Asn Pro
   65                  70                  75                  80
   aac gcc ata gtc act acc att att caa cct ttc aat gct aaa aag gat    288
   Asn Ala Ile Val Thr Thr Ile Ile Gln Pro Phe Asn Ala Lys Lys Asp
                   85                  90                  95
   aag ctt gtt tct tat caa aca ttt gaa gat tct ggt aaa ttg gat tgt    336
   Lys Leu Val Ser Tyr Gln Thr Phe Glu Asp Ser Gly Lys Leu Asp Cys
               100                 105                 110
   gct cca tca tat gct att caa tat gga tcg gac att tcg act ttg acc    384
   Ala Pro Ser Tyr Ala Ile Gln Tyr Gly Ser Asp Ile Ser Thr Leu Thr
           115                 120                 125
   act caa ggt gaa atg tac tac atc tct gct tta tta gat caa ggt tac    432
   Thr Gln Gly Glu Met Tyr Tyr Ile Ser Ala Leu Leu Asp Gln Gly Tyr
       130                 135                 140
   tat gtt gtc act cct gat tac gag ggt cca aag agt aca ttc act gta    480
   Tyr Val Val Thr Pro Asp Tyr Glu Gly Pro Lys Ser Thr Phe Thr Val
   145                 150                 155                 160
   ggg ttg caa tca gga aga gct act ttg aat tcg ctt aga gct act ttg     528
   Gly Leu Gln Ser Gly Arg Ala Thr Leu Asn Ser Leu Arg Ala Thr Leu
                   165                 170                 175
   aaa tca gga aac ttg act ggt gtt tca tca gac gct gag aca tta ttg     576
   Lys Ser Gly Asn Leu Thr Gly Val Ser Ser Asp Ala Glu Thr Leu Leu
               180                 185                 190
   tgg ggt tat tca gga gga agt ctt gct tca gga tgg gct gct gct ata     624
   Trp Gly Tyr Ser Gly Gly Ser Leu Ala Ser Gly Trp Ala Ala Ala Ile
           195                 200                 205
   caa aaa gaa tat gct cca gag ttg agt aaa aac ttg ctt ggt gct gca     672
   Gln Lys Glu Tyr Ala Pro Glu Leu Ser Lys Asn Leu Leu Gly Ala Ala
       210                 215                 220
   ctt ggt gga ttc gtt aca aac att act gcc act gct gaa gct gtt gat     720
   Leu Gly Gly Phe Val Thr Asn Ile Thr Ala Thr Ala Glu Ala Val Asp
   225                 230                 235                 240
   agt ggt cca ttt gca gga atc atc tcc aat gca ttg gct ggt att gga     768
   Ser Gly Pro Phe Ala Gly Ile Ile Ser Asn Ala Leu Ala Gly Ile Gly
                   245                 250                 255
   aat gaa tac cct gat ttc aaa aac tat ctt ttg aaa aaa gtg tca cca     816
   Asn Glu Tyr Pro Asp Phe Lys Asn Tyr Leu Leu Lys Lys Val Ser Pro
               260                 265                 270
   ttg ctt tca atc act tat cgt ttg gga aac act cac tgt ttg ctt gat     864
   Leu Leu Ser Ile Thr Tyr Arg Leu Gly Asn Thr His Cys Leu Leu Asp
           275                 280                 285
   ggt ggt att gct tat ttc ggt aaa tca ttc ttt tcc aga att att aga     912
   Gly Gly Ile Ala Tyr Phe Gly Lys Ser Phe Phe Ser Arg Ile Ile Arg
       290                 295                 300
   tat ttc cct gat gga tgg gat ctt gtc aac caa gaa cct atc aaa acc     960
   Tyr Phe Pro Asp Gly Trp Asp Leu Val Asn Gln Glu Pro Ile Lys Thr
   305                 310                 315                 320
   atc ttg caa gat aat gga ttg gtt tac caa cca aag gac ttg acc cca     1008
   Ile Leu Gln Asp Asn Gly Leu Val Tyr Gln Pro Lys Asp Leu Thr Pro
                   325                 330                 335
   caa att cca tta ttc atc tac cac ggt acc ttg gat gca att gtc ccc     1056
   Gln Ile Pro Leu Phe Ile Tyr His Gly Thr Leu Asp Ala Ile Val Pro
               340                 345                 350
   att gtc aac tca aga aag aca ttc caa caa tgg tgt gat tgg gga ctc     1104
   Ile Val Asn Ser Arg Lys Thr Phe Gln Gln Trp Cys Asp Trp Gly Leu
           355                 360                 365
   aaa tct ggt gaa tat aat gaa gat ttg acc aat gga cac att act gaa     1152
   Lys Ser Gly Glu Tyr Asn Glu Asp Leu Thr Asn Gly His Ile Thr Glu
       370                 375                 380
   tca att gtg ggt gca cca gct gct ttg act tgg att atc aat cgt ttc     1200
   Ser Ile Val Gly Ala Pro Ala Ala Leu Thr Trp Ile Ile Asn Arg Phe
   385                 390                 395                 400
   aat gga cag cct cca gtt gat gga tgt caa cat aat gtg aga gct tca     1248
   Asn Gly Gln Pro Pro Val Asp Gly Cys Gln His Asn Val Arg Ala Ser
                   405                 410                 415
   aac ttg gaa tat cca gga act cca caa tca atc aag aat tac ttt gaa    1296
   Asn Leu Glu Tyr Pro Gly Thr Pro Gln Ser Ile Lys Asn Tyr Phe Glu
               420                 425                 430
   gct gca ttg cac gca att ttg ggc ttt gat ttg ggt cca gat gtt aag    1344
   Ala Ala Leu His Ala Ile Leu Gly Phe Asp Leu Gly Pro Asp Val Lys
           435                 440                 445
   aga gat aag gtt act ttg ggc gga ttg ctc aag ttg gaa cgt ttt gct    1392
   Arg Asp Lys Val Thr Leu Gly Gly Leu Leu Lys Leu Glu Arg Phe Ala
       450                 455                 460
   ttt cat cat cat cat cat cat taa                                    1416
   Phe His His His His His His
   465                 470
<210> 4
   <211> 471
   <212> PRT
   <213> Candida parapsilosis
<400> 4
   Met Arg Tyr Phe Ala Ile Ala Phe Leu Leu Ile Asn Thr Ile Ser Ala
   1               5                   10                  15
   Phe Val Leu Ala Pro Lys Lys Pro Ser Gln Asp Asp Phe Tyr Thr Pro
               20                  25                  30
   Pro Gln Gly Tyr Glu Ala Gln Pro Leu Gly Ser Ile Leu Lys Thr Arg
           35                  40                  45
   Asn Val Pro Asn Pro Leu Thr Asn Val Phe Thr Pro Val Lys Val Gln
       50                  55                  60
   Asn Ala Trp Gln Leu Leu Val Arg Ser Glu Asp Thr Phe Gly Asn Pro
   65                  70                  75                  80
   Asn Ala Ile Val Thr Thr Ile Ile Gln Pro Phe Asn Ala Lys Lys Asp
                   85                  90                  95
   Lys Leu Val Ser Tyr Gln Thr Phe Glu Asp Ser Gly Lys Leu Asp Cys
               100                 105                 110
   Ala Pro Ser Tyr Ala Ile Gln Tyr Gly Ser Asp Ile Ser Thr Leu Thr
           115                 120                 125
   Thr Gln Gly Glu Met Tyr Tyr Ile Ser Ala Leu Leu Asp Gln Gly Tyr
       130                 135                 140
   Tyr Val Val Thr Pro Asp Tyr Glu Gly Pro Lys Ser Thr Phe Thr Val
   145                 150                 155                 160
   Gly Leu Gln Ser Gly Arg Ala Thr Leu Asn Ser Leu Arg Ala Thr Leu
                   165                 170                 175
   Lys Ser Gly Asn Leu Thr Gly Val Ser Ser Asp Ala Glu Thr Leu Leu
               180                 185                 190
   Trp Gly Tyr Ser Gly Gly Ser Leu Ala Ser Gly Trp Ala Ala Ala Ile
           195                 200                 205
   Gln Lys Glu Tyr Ala Pro Glu Leu Ser Lys Asn Leu Leu Gly Ala Ala
       210                 215                 220
   Leu Gly Gly Phe Val Thr Asn Ile Thr Ala Thr Ala Glu Ala Val Asp
   225                 230                 235                 240
   Ser Gly Pro Phe Ala Gly Ile Ile Ser Asn Ala Leu Ala Gly Ile Gly
                   245                 250                 255
   Asn Glu Tyr Pro Asp Phe Lys Asn Tyr Leu Leu Lys Lys Val Ser Pro
               260                 265                 270
   Leu Leu Ser Ile Thr Tyr Arg Leu Gly Asn Thr His Cys Leu Leu Asp
           275                 280                 285
   Gly Gly Ile Ala Tyr Phe Gly Lys Ser Phe Phe Ser Arg Ile Ile Arg
       290                 295                 300
   Tyr Phe Pro Asp Gly Trp Asp Leu Val Asn Gln Glu Pro Ile Lys Thr
   305                 310                 315                 320
   Ile Leu Gln Asp Asn Gly Leu Val Tyr Gln Pro Lys Asp Leu Thr Pro
                   325                 330                 335
   Gln Ile Pro Leu Phe Ile Tyr His Gly Thr Leu Asp Ala Ile Val Pro
               340                 345                 350
   Ile Val Asn Ser Arg Lys Thr Phe Gln Gln Trp Cys Asp Trp Gly Leu
           355                 360                 365
   Lys Ser Gly Glu Tyr Asn Glu Asp Leu Thr Asn Gly His Ile Thr Glu
       370                 375                 380
   Ser Ile Val Gly Ala Pro Ala Ala Leu Thr Trp Ile Ile Asn Arg Phe
   385                 390                 395                 400
   Asn Gly Gln Pro Pro Val Asp Gly Cys Gln His Asn Val Arg Ala Ser
                   405                 410                 415
   Asn Leu Glu Tyr Pro Gly Thr Pro Gln Ser Ile Lys Asn Tyr Phe Glu
               420                 425                 430
   Ala Ala Leu His Ala Ile Leu Gly Phe Asp Leu Gly Pro Asp Val Lys
           435                 440                 445
   Arg Asp Lys Val Thr Leu Gly Gly Leu Leu Lys Leu Glu Arg Phe Ala
       450                 455                 460
   Phe His His His His His His
   465                 470